# EUROPÄISCHE PATENTSCHRIFT

(11) **EP 1 119 559 B1**
(45) Veröffentlichungstag und Bekanntmachung des Hinweises auf die Patenterteilung: **26.11.2003**
(21) Anmeldenummer: 99950547.2
(22) Anmeldetag: 24.09.1999
(51) Int. Cl.: C07D 309/36, C07D 405/04, A01N 43/16

(54) **3-PHENYL-PYRONE**
3-PHENYL-PYRONES
3-PHENYL-PYRONES

(30) Priorität: 09.10.1998 DE 19846517
(43) Veröffentlichungstag der Anmeldung: 01.08.2001
(73) Patentinhaber: Bayer CropScience AG, 40789 Monheim (DE)
(72) Erfinder: LIEB, Folker, D-51375 Leverkusen (DE); FISCHER, Reiner, D-40789 Monheim (DE); GRAFF, Alan, D-51061 Köln (DE); SCHNEIDER, Udo, D-51373 Leverkusen (DE); RUTHER, Michael, D-40764 Langenfeld (DE); ERDELEN, Christoph, D-42799 Leichlingen (DE); ANDERSCH, Wolfram, D-51469 Bergisch Gladbach (DE); WACHENDORFF-NEUMANN, Ulrike, D-56566 Neuwied (DE); HÄNSSLER, Gerd, D-51381 Leverkusen (DE); MAULER-MACHNIK, Astrid, D-42799 Leichlingen (DE); STENZEL, Klaus, D-40595 Düsseldorf (DE)
(86) Internationale Anmeldenummer: EP9907113
(87) Internationale Veröffentlichungsnummer: WO00021946

(56) Entgegenhaltungen:
- EP-A- 0 588 137

## Beschreibung

Die vorliegende Erfindung betrifft neue 3-Phenyl-pyrone, ein Verfahren zu deren Herstellung und deren Verwendung als Pestizide, Fungizide und Herbizide.

3-Aryl-pyron-Derivate und ihre Verwendung als Schädlingsbekämpfungsmittel sind bekannt (vgl. EP-A-588 137).

Weiterhin sind bereits zahlreiche Oxymethoxy-3-phenyl-pyron-Derivate mit pestiziden, fungiziden und herbiziden Eigenschaften bekannt geworden (vgl. WO 97-19 941). Die Wirksamkeit dieser Stoffe ist gut, läßt aber bei niedrigen Aufwandmengen beziehungsweise bei bestimmten Indikationen in manchen Fällen zu wünschen übrig.

Weiterhin sind 3-(2-Methyl-4-fluor-phenyl)-4-hydroxy-5-methyl-6-(pyrid-2-yl)-pyron und 3-(2-Methyl-4-chlor-phenyl)-4-hydroxy-5-methyl-6-(4-fluor-phenyl)-pyron schon als Zwischenprodukte beschrieben worden (vgl. WO 97-19 941). Biologische Eigenschaften dieser Substanzen wurden allerdings bisher noch nicht erwähnt.

Es wurden nun neue 3-Phenyl-pyrone der Formel 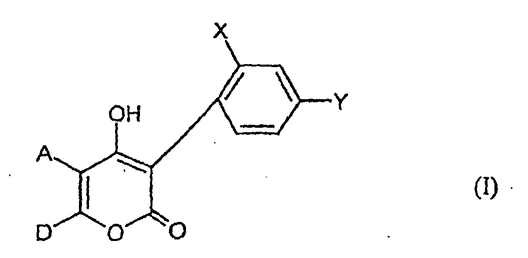 in welcher
- X: für Alkyl mit 1 bis 6 Kohlenstoffatomen steht und
- Y: für Fluor, Chlor oder Brom steht
oder
- X: für Fluor, Chlor oder Brom steht und
- Y: für Alkyl mit 1 bis 6 Kohlenstoffatomen steht.
- A: steht bevorzugt für Wasserstoff, Alkyl mit 1 bis 12 Kohlenstoffatomen oder für Phenyl, das einfach bis dreifach, gleichartig oder verschieden substituiert sein kann durch Halogen, C₁-C₆-Alkyl, C₁-C₆-Halagenalkyl, C₁-C₆-Alkoxy, C₁-C₆-Halogenalkoxy, Cyano und/oder Nitro.
- D: steht bevorzugt für Wasserstoff, Alkyl mit 1 bis 12 Kohlenstoffatomen, für gegebenenfalls durch Halogen, Alkyl mit 1 bis 4 Kohlenstoffatomen, Alkoxy mit 1 bis 4 Kohlenstoffatomen und/oder Halogenalkyl mit 1 bis 4 Kohlenstoffatomen substituiertes Cycloalkyl mit 3 bis 8 Kohlenstoffatomen,
oder für Phenyl, das einfach bis dreifach, gleichartig oder verschieden substituiert sein kann durch Halogen, C₁-C₆-Alkyl, C₁-C₆-Halogenalkyl, C₁-C₆-Alkoxy, C₁-C₆-Halogenalkoxy, Cyano, Nitro, Phenyl und/oder Phenoxy, wobei die beiden letztgenannten Reste ihrerseits einfach oder zweifach substituiert sein können durch Halogen, C₁-C₄-Alkyl, C₁-C₄-Alkoxy, C₁-C₄-Halogenalkyl und/oder C₁-C₄-Halogenalkoxy,
oder für fünf- oder sechsgliedriges Heteroaryl mit 1 oder 2 Heteroatomen aus der Reihe Sauerstoff, Schwefel und Stickstoff, wobei diese Reste einfach oder zweifach substituiert sein können durch Halogen, C₁-C₆-Alkyl, C₁-C₆-Halogenalkyl, C₁-C₆-Alkoxy, C₁-C₆-Halogenalkoxy, Cyano und/oder Nitro,
oder für einen Rest der Formel 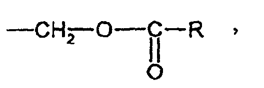 worin
R bevorzugt für Phenyl steht, das einfach bis dreifach, gleichartig oder verschieden substituiert sein kann durch Halogen, C₁-C₆-Alkyl, C₁-C₆-Halogenalkyl, C₁-C₆-Alkoxy, C₁-C₆-Halogenalkoxy, Cyano und/oder Nitro.
- A und D: stehen außerdem auch gemeinsam bevorzugt für eine C₃-C₆-Alkandiyl-, C₃-C₆-Alkendiyl- oder C₄-C₆-Alkadiendiylgruppe, worin jeweils gegebenenfalls eine Methylengruppe durch Sauerstoff oder Schwefel ersetzt ist, und welche jeweils gegebenenfalls substituiert sind durch Halogen, C₁-C₆-Alkyl, C₁-C₆-Halogenalkyl, C₁-C₆-Alkoxy, C₁-C₆-Halogenalkoxy, oder durch eine weitere, einen ankondensierten Ring bildende C₃-C₆-Alkandiyl-, C₃-C₆-Alkendiyl- oder C₄-C₆-Alkadiendiylgruppe, in welchen gegebenenfalls jeweils eine Methylengruppe durch Sauerstoff oder Schwefel ersetzt ist und welche gegebenenfalls durch C₁-C₆-Alkyl substituiert sind.

Die Verbindungen der Formel (I) können in Abhängigkeit von der Art der Substituenten als geometrische und/oder optische Isomere oder Isomerengemische, in unterschiedlicher Zusammensetzung vorliegen, die gegebenenfalls in üblicher Art und Weise getrennt werden können. Die vorliegende Erfindung betrifft sowohl die reinen Isomeren als auch Isomerengemische. Im folgenden wird der Einfachheit halber jedoch stets von 3-Phenyl-pyronen der Formel (I) gesprochen, obwohl damit sowohl die reinen Isomeren als gegebenenfalls auch Gemische mit unterschiedlichen Anteilen an Isomeren gemeint sind.

Die 3-Phenyl-pyrone der Formel (I) können durch die Formel 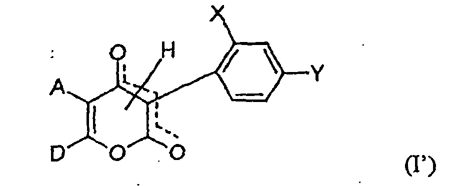 bildlich dargestellt werden. Die gestrichelte Linie soll andeuten, daß diese Stoffe in den beiden tautomeren Formen 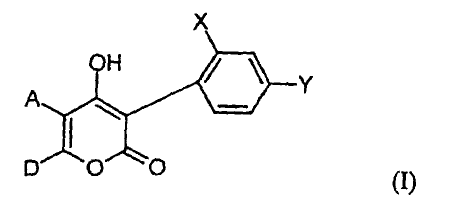 und 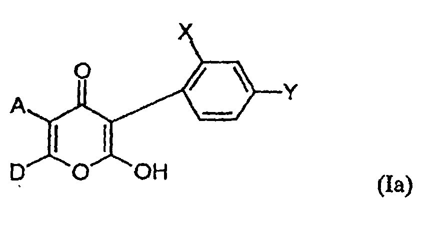 vorhanden sein können.

Die erfindungsgemäßen 3-Phenyl-pyrone können entweder als Gemische von Verbindungen der Formeln (I) und (Ia) oder auch in Form von reinen Isomeren vorliegen. Gemische von Verbindungen der Formeln (I) und (Ia) lassen sich auf physikalischem Wege, zum Beispiel durch chromatographische Methoden, trennen.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Weiterhin wurde gefunden, daß sich 3-Phenyl-pyrone der Formel (I) herstellen lassen, indem man
- α): entweder Carbonyl-Verbindungen der Formel 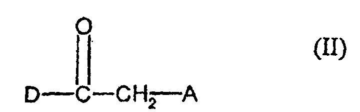
in welcher
A und D die oben angegebenen Bedeutungen haben,
oder
- β): Silylether der Formel 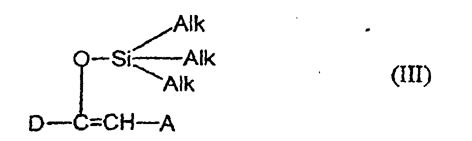 in welcher
A und D die oben angegebenen Bedeutungen haben und
Alk für Alkyl mit 1 bis 4 Kohlenstoffatomen steht,
jeweils mit Keten-Derivaten der Formel 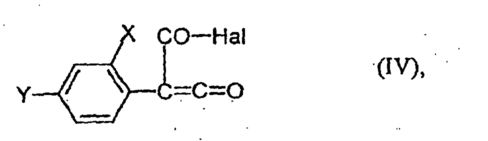 in welcher
X und Y die oben angegebenen Bedeutungen haben und
- Hal: für Chlor oder Brom steht,
gegebenenfalls in Gegenwart eines Verdünnungsmittels und gegebenenfalls in Gegenwart eines Säureakzeptors umsetzt.

Schließlich wurde gefunden, daß die erfindungsgemäßen 3-Phenyl-pyrone der Formel (I) eine sehr gute pestizide, fungizide und herbizide Wirksamkeit besitzen.

Überraschenderweise eignen sich die erfindungsgemäßen 3-Phenyl-pyrone der Formel (I) besser als Pestizide, Fungizide und Herbizide als die konstitutionell ähnlichsten, vorbekannten Wirkstoffe gleicher Wirkungsrichtung.

Die erfindungsgemäßen 3-Phenyl-pyrone sind durch die Formel (I) allgemein definiert.

In den Verbindungen der Formel (I) stehen besonders bevorzugt
- X: für Alkyl mit 1 bis 4 Kohlenstoffatomen und
- Y: für Fluor, Chlor oder Brom
oder
- X: für Chlor oder Brom und
- Y: für Alkyl mit 1 bis 4 Kohlenstoffatomen.
- A: steht besonders bevorzugt für Wasserstoff, Alkyl mit 1 bis 8 Kohlenstoffatomen oder für Phenyl, das einfach bis dreifach, gleichartig oder verschieden substituiert sein kann durch Fluor, Chlor, Brom, C₁-C₄-Alkyl, C₁-C₄-Halogenalkyl, C₁-C₄-Alkoxy, C₁-C₄-Halogenalkoxy, Cyano und/oder Nitro.
- D: steht besonders bevorzugt für Wasserstoff, Alkyl mit 1 bis 10 Kohlenstoffatomen, für gegebenenfalls einfach bis dreifach, gleichartig oder verschieden durch Fluor, Chlor C₁-C₃-Alkyl, C₁-C₂-Alkoxy, C₁-C₂-Halogenalkyl substituiertes Cycloalkyl mit 3 bis 7 Kohlenstoffatomen,
oder für Phenyl, das einfach bis dreifach, gleichartig oder verschieden substituiert sein kann durch Fluor, Chlor, Brom, C₁-C₄-Alkyl, C₁-C₄-Halogenalkyl, C₁-C₄-Alkoxy, C₁-C₄-Halogenalkoxy, Cyano, Nitro, Phenyl und/oder Phenoxy, wobei die beiden letztgenannten Reste ihrerseits einfach oder zweifach substituiert sein können durch Fluor, Chlor, Brom, Methyl, Ethyl, Methoxy, Trifluormethyl, Trifluormethoxy und/oder Difluormethoxy,
oder für Furanyl, Pyridyl, Imidazolyl, Triazolyl, Pyrazolyl, Pyrimidinyl, Thiazolyl oder Thienyl, wobei diese Reste einfach oder zweifach substituiert sein können durch Fluor, Chlor, Brom, C₁-C₄-Alkyl, C₁-C₄-Halogenalkyl, C₁-C₄-Alkoxy, C₁-C₄-Halogenalkoxy, Cyano und/oder Nitro,
oder
- D: steht besonders bevorzugt für einen Rest der Formel 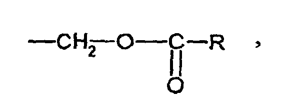 worin
R besonders bevorzugt für Phenyl steht, das einfach bis dreifach, gleichartig oder verschieden substituiert sein kann durch Fluor, Chlor, Brom, C₁-C₄-Alkyl, C₁-C₄-Halogenalkyl, C₁-C₄-Alkoxy, C₁-C₄-Halogenalkoxy, Cyano und/oder Nitro.
- A und D: stehen außerdem gemeinsam besonders bevorzugt für eine C₃-C₅-Alkandiyl oder C₃-C₅-Alkendiylgruppe, worin jeweils gegebenenfalls ein Kohlenstoffatom durch Sauerstoff oder Schwefel ersetzt ist, und welche gegebenenfalls substituiert sind durch Fluor, Chlor, Brom, C₁-C₄-Alkyl, C₁-C₄-Alkoxy, C₁-C₄-Halogenalkyl und/oder C₁-C₆-Halogenalkoxy, oder durch eine weitere, einen ankondensierten Ring bildende C₄-Alkandiendiylgruppe, die durch C₁-C₄-Alkyl substituiert sein kann.

In den Verbindungen der Formel (I) stehen ganz besonders bevorzugt
- X: für Methyl oder Ethyl und
- Y: für Fluor oder Chlor
oder
- X: für Chlor oder Brom und
- Y: für Methyl oder Ethyl.
- A: steht ganz besonders bevorzugt für Wasserstoff, Alkyl mit 1 bis 6 Kohlenstoffatomen oder für Phenyl, das einfach oder zweifach, gleichartig oder verschieden substituiert sein kann durch Fluor, Chlor, Methyl, Ethyl, n-Propyl, Isopropyl, Trifluormethyl, Methoxy, Ethoxy, Trifluormethoxy, Cyano und/oder Nitro.
- D: steht ganz besonders bevorzugt für Wasserstoff, Alkyl mit 1 bis 8 Kohlenstoffatomen,
oder für gegebenenfalls einfach oder zweifach, gleichartig oder verschieden durch Fluor, Chlor, Methyl, Ethyl, Methoxy, Ethoxy und/oder Trifluormethyl substituiertes Cycloalkyl mit 3 bis 7 Kohlenstoffatomen,
oder für Phenyl, das einfach oder zweifach, gleichartig oder verschieden substituiert sein kann durch Fluor, Chlor, Brom, Methyl, Ethyl, n-Propyl, Isopropyl, n-Butyl, sek.-Butyl, iso-Butyl, tert.-Butyl, Trifluormethyl, Methoxy, Ethoxy, Trifluormethoxy, Cyano, Nitro, Phenyl und/oder Phenoxy, wobei die beiden letztgenannten Reste ihrerseits einfach oder zweifach substituiert sein können durch Fluor, Chlor, Methyl, Methoxy, Trifluormethyl und/oder Trifluormethoxy,
oder für Furanyl, Pyridyl oder Thienyl, wobei diese Reste einfach oder zweifach substituiert sein können durch Fluor, Chlor, Brom, Methyl, Ethyl, n-Propyl, Isopropyl, Trifluormethyl, Trifluormethoxy, Cyano und/oder Nitro,
oder
- D: steht ganz besonders bevorzugt für einen Rest der Formel 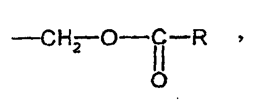 worin
R besonders bevorzugt für Phenyl steht, das einfach oder zweifach, gleichartig oder verschieden substituiert sein kann durch Fluor, Chlor, Methyl, Ethyl, Trifluormethyl, Methoxy, Trifluormethoxy, Cyano und/oder Nitro.
- A und D: stehen außerdem gemeinsam ganz besonders bevorzugt für eine C₃-C₅-Alkandiyl oder C₃-C₅-Alkendiylgruppe, worin jeweils gegebenenfalls eine Methylengruppe durch Sauerstoff oder Schwefel ersetzt ist, und welche gegebenenfalls einfach bis vierfach, gleichartig oder verschieden substituiert ist durch Fluor, Chlor, Brom, Methyl, Ethyl, n-Propyl, Isopropyl n-Butyl, iso-Butyl, sek.-Butyl, tert.-Butyl, Methoxy, Trifluormethyl und/oder Trifluormethoxy, oder durch eine weitere, einen ankondensierten Ring bildende Butadiendiylgruppe, die einfach oder zweifach durch Methyl substituiert sein kann.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend. Außerdem können auch einzelne Definitionen entfallen.

Ausgenommen von den erfindungsgemäßen 3-Phenyl-pyronen sind, jeweils die Verbindungen der Formeln

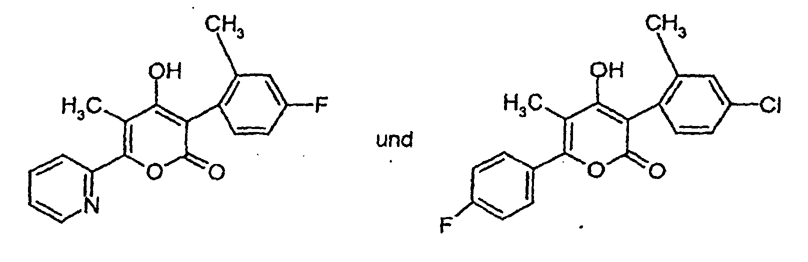

Im einzelnen seien außer den in den Herstellungsbeispielen genannten 3-Phenyl-pyronen die folgenden Stoffe der Formel (I) genannt.

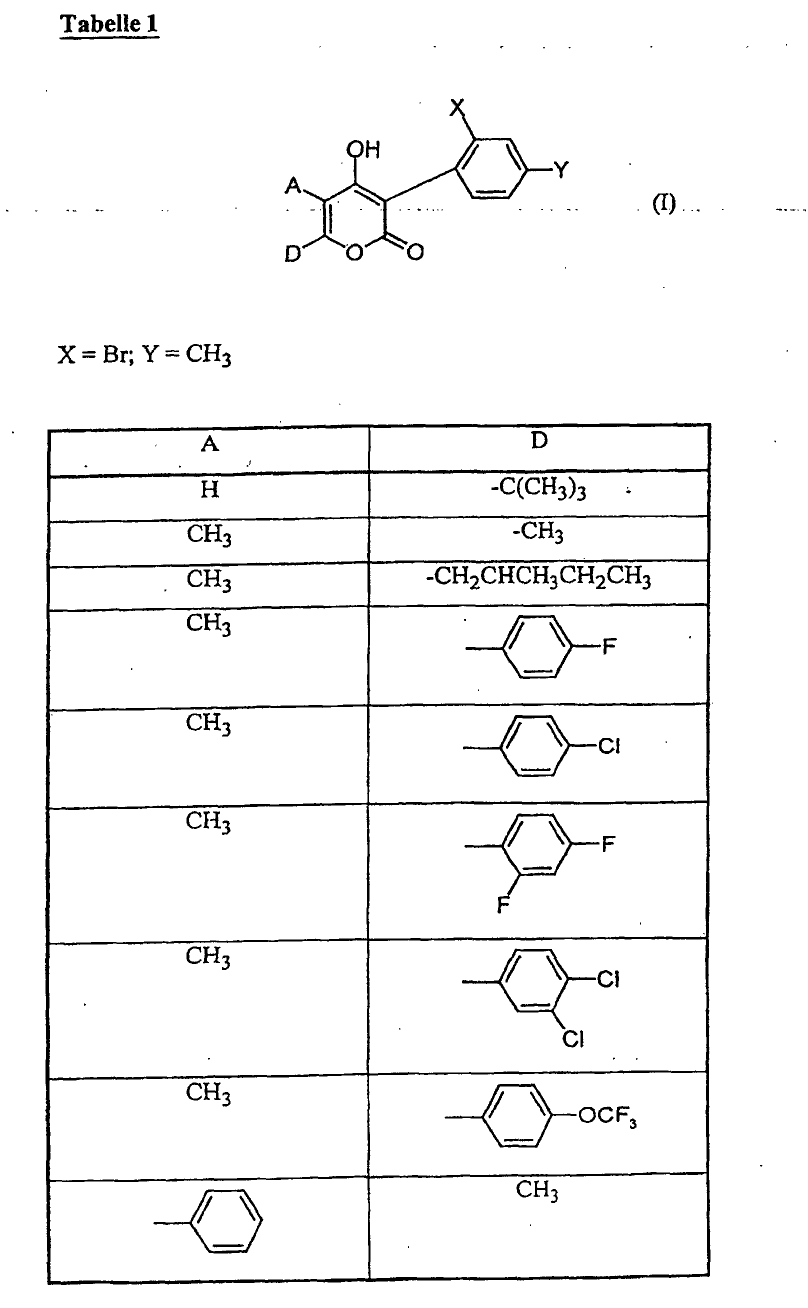

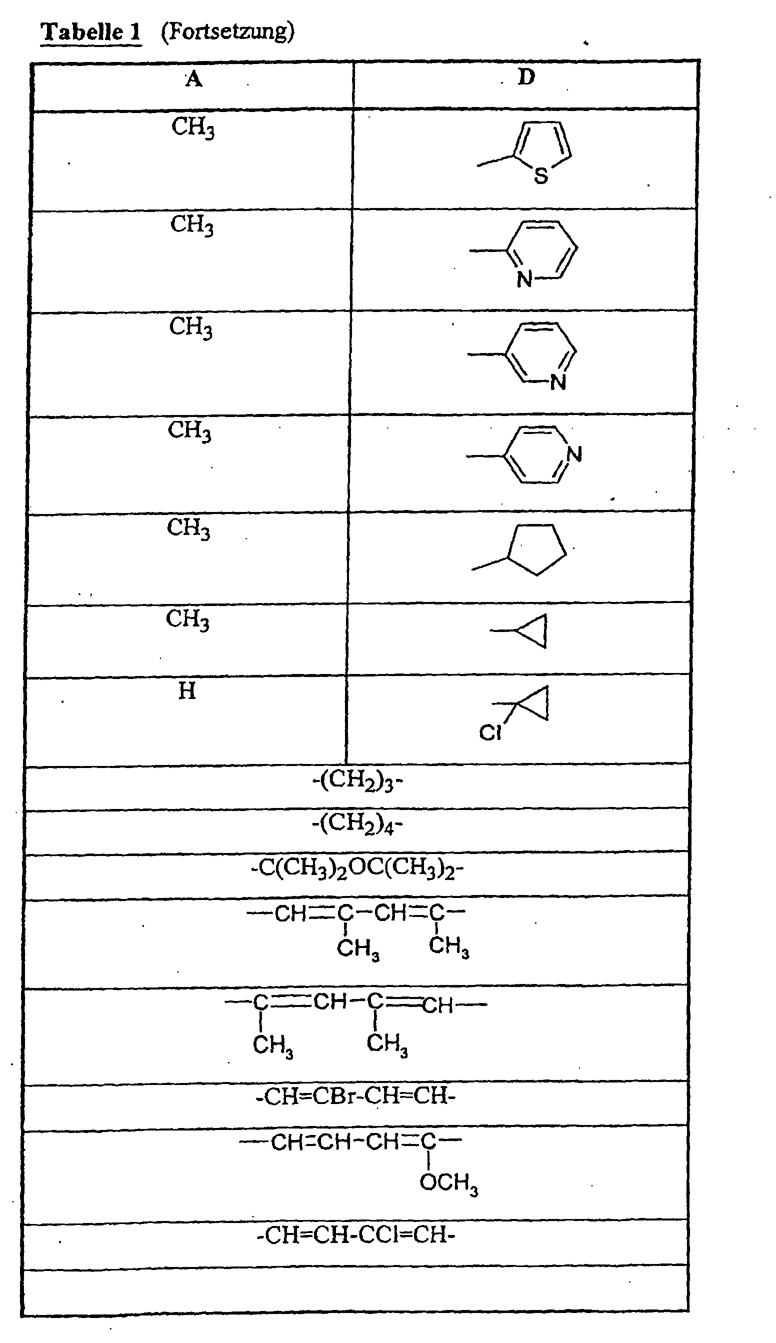

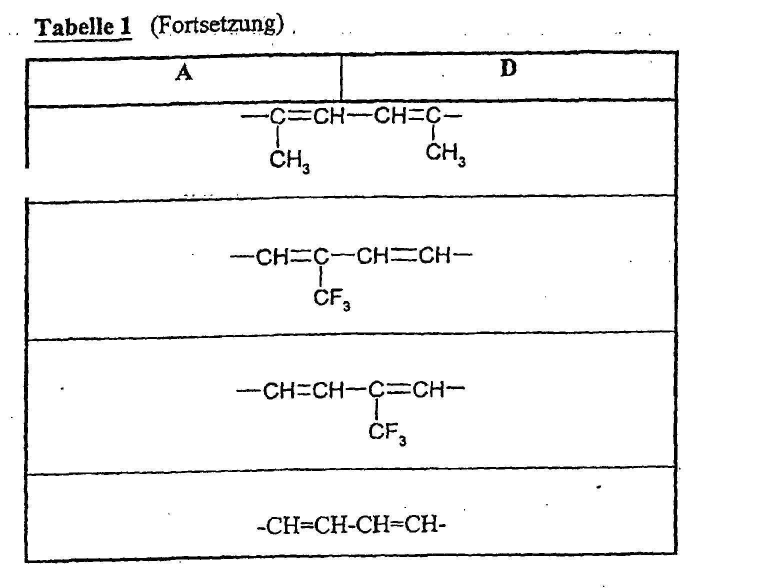

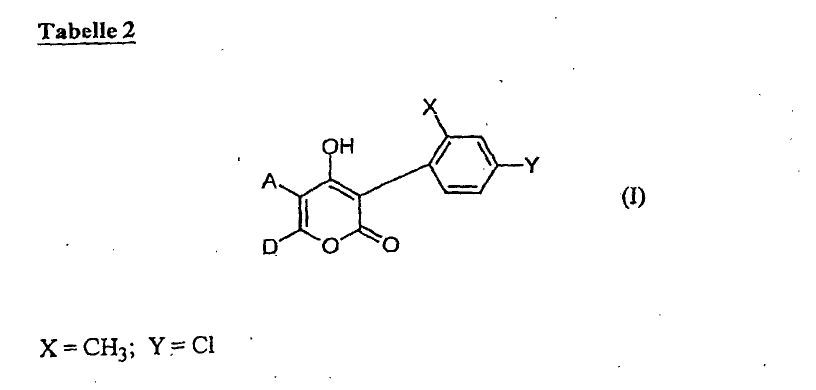

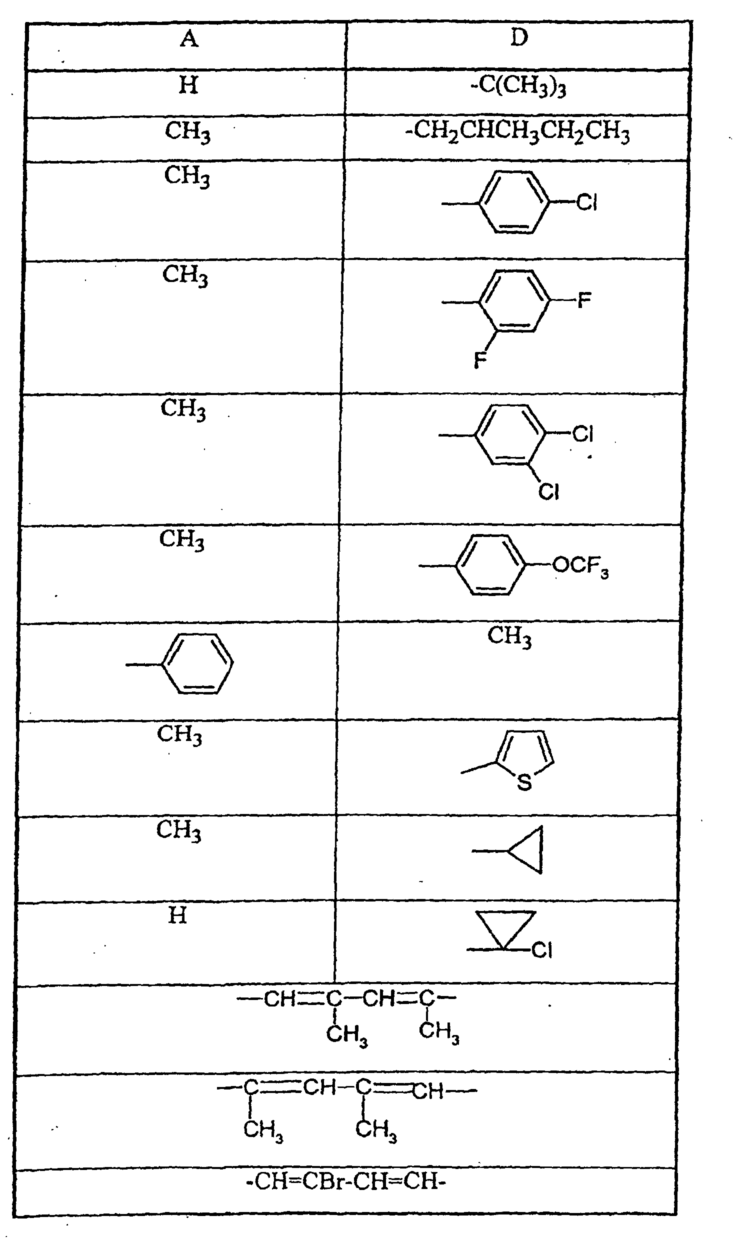

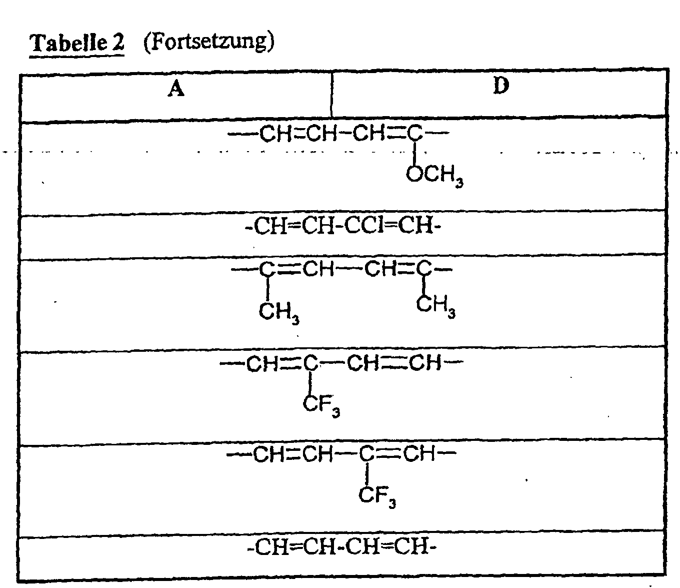

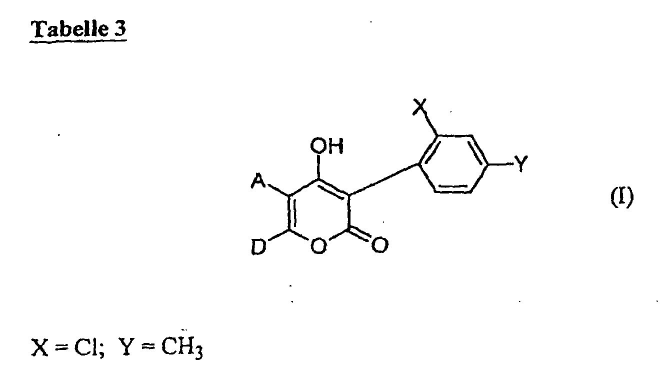

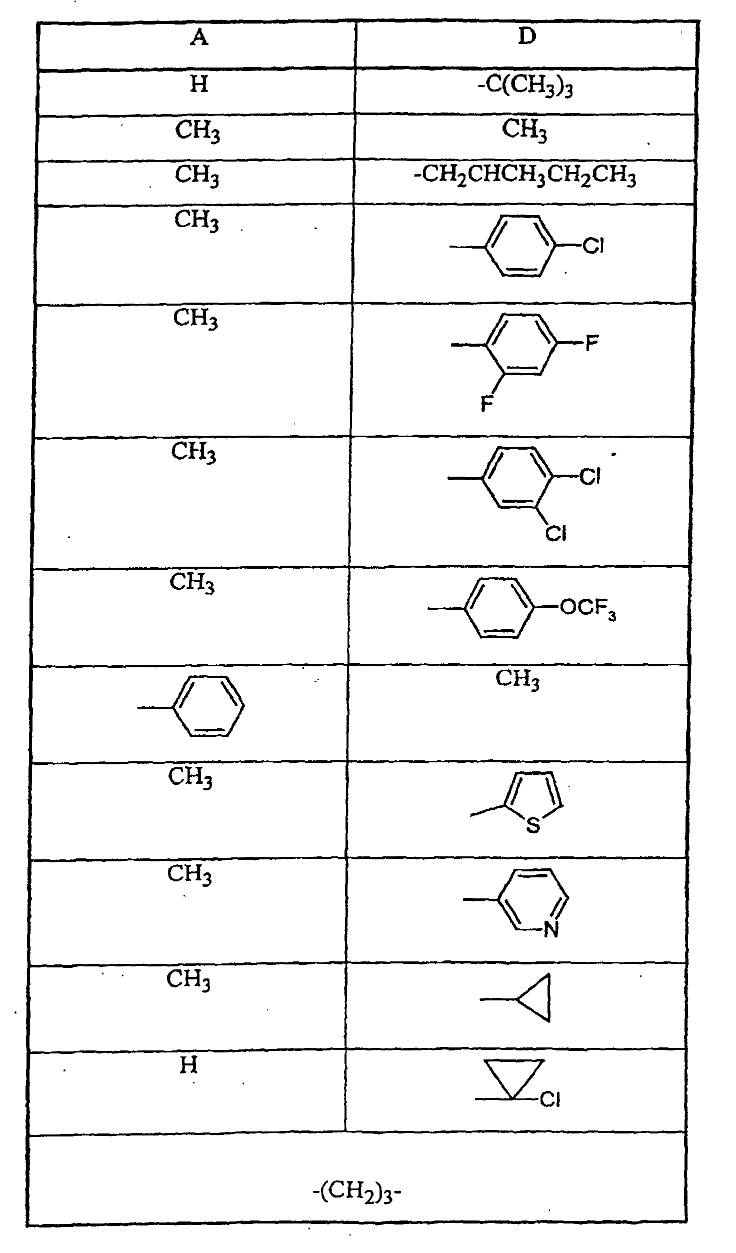

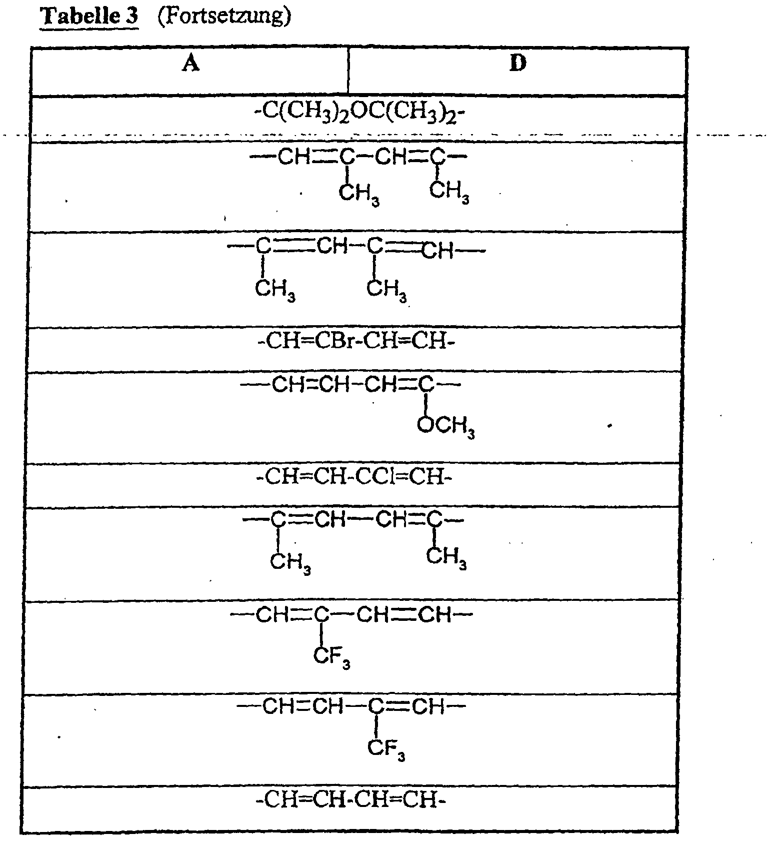

Verwendet man (Chlorcarbonyl)-2-(2-methyl-4-chlor-phenyl)-keten und Ethyl-(pyrid-2-yl)-keton als Ausgangssubstanzen, so kann der Verlauf des erfindungsgemäßen Verfahrens (Variante α) durch das folgende Formelschema veranschaulicht werden.

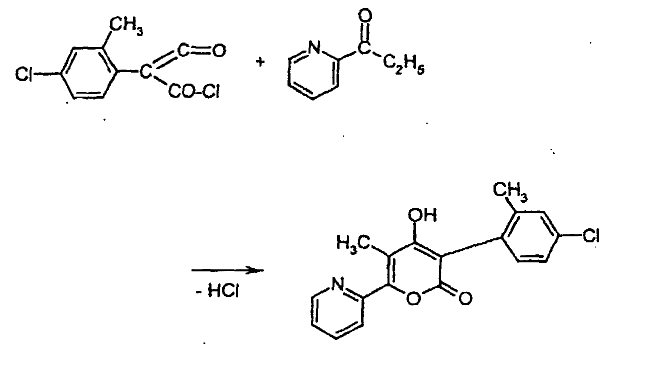

Verwendet man (Chlore-arbonyl)-2-(2-methyl-4-chlor-phenyl)-keten und 1-(Pyrid-2-yl)-**1**-trimethyl-silyloxy-prop-1-en als Ausgangssubstanzen, so kann der Verlauf des erfindungsgemäßen Verfahrens (Variante β) durch das folgende Formelschema veranschaulicht werden.

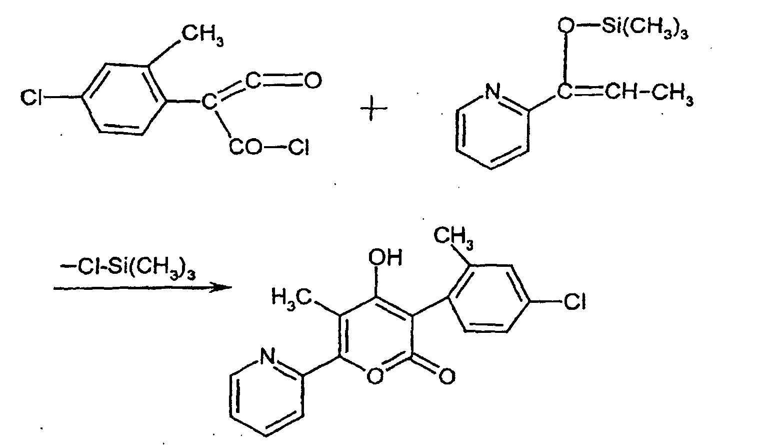

Die bei der Durchführung des erfindungsgemäßen Verfahrens nach Variante α als Ausgangsstoffe benötigten Carbonyl-Verbindungen sind durch die Formel (II) allgemein definiert. In dieser Formel haben A und D vorzugsweise diejenigen Bedeutungen, die bereits im Zusammenhang mit der Beschreibung der erfindungsgemäßen 3-Phenyl-pyrone der Formel (I) für diese Reste als bevorzugt genannt wurden.

Die Carbonyl-Verbindungen der Formel (II) sind bekannt oder lassen sich nach bekannten Verfahren herstellen.

Die bei der Durchführung des erfindungsgemäßen Verfahrens nach Variante β als Ausgangsstoffe benötigten Silylether sind durch die Formel (III) allgemein definiert. In dieser Formel haben A und D vorzugsweise diejenigen Bedeutungen, die bereits im Zusammenhang mit der Beschreibung der erfindungsgemäßen 3-Phenyl-pyrone der Formel (I) für diese Reste als bevorzugt genannt wurden. Alk steht vorzugsweise für Methyl oder Ethyl, besonders bevorzugt für Methyl.

Die Silylether der Formel (III) sind bekannt oder lassen sich nach bekannten Methoden herstellen.

Die bei der Durchführung des erfindungsgemäßen Verfahrens als Reaktionskomponenten benötigten Keten-Derivate sind durch die Formel (IV) allgemein definiert. In dieser Formel haben X und Y vorzugsweise diejenigen Bedeutungen, die bereits im Zusammenhang mit der Beschreibung der erfindungsgemäßen 3-Phenyl-pyrone der Formel (I) für diese Reste als bevorzugt genannt wurden. Hal steht auch vorzugsweise für Chlor oder Brom.

Die Keten-Derivate der Formel (IV) sind bekannt oder lassen sich nach bekannten Verfahren herstellen (vgl. Org. Prep. Proced. Int. 7, 155-158 (1975) und DE-A 1 945 703). So erhält man Keten-Derivate der Formel (IV), indem man
substituierte Phenylmalonsäuren der Formel 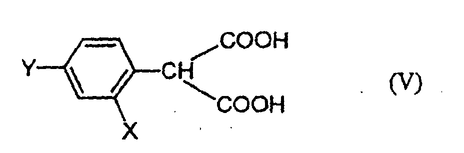 in welcher
X und Y die oben angegebenen Bedeutungen haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Diethylformamid, Methyl-Sterylformamid oder Triphenylphosphin und gegebenenfalls in Gegenwart von Basen wie z.B. Pyridin oder Triethylamin, bei einer Temperatur zwischen -20°C und +200°C, bevorzugt zwischen 0°C und 150°C, umsetzt.

Die substituierten Phenylmalonsäuren der Formel (V) sind bekannt oder lassen sich nach bekannten Methoden herstellen (vgl. z.B. Organikum, VEB Deutscher Verlag der Wissenschaften, Berlin 1977, S. 517 ff). So erhält man substituierte Phenylmalonsäuren der Formel (V), indem man substituierte Phenylmalonsäureester der Formel 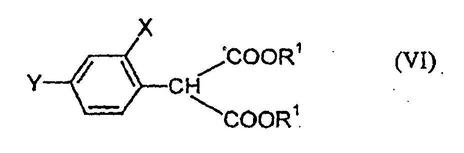 in welcher
- X und Y: die oben angegebenen Bedeutungen haben und
- R¹: für Alkyl mit 1 bis 4 Kohlenstoffatomen steht,
mit Alkalimetallhydroxiden, wie Natriumhydroxid oder Kaliumhydroxid, in Gegenwart eines Verdünnungsmittels, wie Wasser, bei Temperaturen zwischen 0°C und 30°C umsetzt.

In der Formel (VI) haben X und Y vorzugsweise diejenigen Bedeutungen, die bereits im Zusammenhang mit der Beschreibung der erfindungsgemäßen 3-Phenyl-pyrone der Formel (I) für diese Reste als bevorzugt genannt wurden. R¹ steht vorzugsweise für Methyl oder Ethyl.

Die substituierten Phenylmalonsäureester der Formel (VI) sind bekannt oder lassen sich nach bekannten Methoden herstellen (vgl. Tetrahedron Letters 27, 2763 (1986) und Organikum, VEB Deutscher Verlag der Wissenschaften, Berlin 1977. S. 587 ff.).

Als Verdünnungsmittel kommen bei der Durchführung des erfindungsgemäßen Verfahrens nach den Varianten α und β alle üblichen, gegenüber den Reaktionsteilnehmern inerten organischen Solventien in Frage. Vorzugsweise verwendbar sind Kohlenwasserstoffe, wie o-Dichlorbenzol, Tetralin, Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid oder N-Methylpyrrolidon.

Als Säureakzeptoren kommen bei der Durchführung des erfindungsgemäßen Verfahrens nach den Varianten α und β alle üblichen Säurebindemittel in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base oder N,N-Dimethyl-anilin.

Die Reaktionstemperaturen können bei der Durchführung des erfindungsgemäßen Verfahrens nach den Varianten α und β innerhalb eines größeren Bereiches variiert werden. Zweckmäßigerweise arbeitet man bei Temperaturen zwischen 0°C und 250°C, vorzugsweise zwischen 50°C und 220°C.

Bei der Durchführung des erfindungsgemäßen Verfahrens nach dem Varianten α und β arbeitet man vorzugsweise unter Atmosphärendruck.

Bei der Durchführung des erfindungsgemäßen Verfahrens setzt man auf 1 Mol an Carbonyl-Verbindung der Formel (II) beziehungsweise auf 1 Mol an Silylether der Formel (III) im allgemeinen eine äquimolare Menge an Keten-Derivat der Formel (IV) und gegebenenfalls auch eine äquimolare Menge an Säureakzeptor ein. Es ist jedoch auch möglich, die eine oder andere Komponente in einem größeren Überschuß (bis zu 5 Mol) zu verwenden.

Die erfindungsgemäßen 3-Phenyl-pyrone der Formel (I) besitzen eine sehr gute pestizide Wirksamkeit und weisen gegenüber Kulturpflanzen eine sehr gute Verträglichkeit auf.

Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus
   Aus der Ordnung der Chilopoda z.B. Geophilus carpophagus, Scutigera spec.
   Aus der Ordnung der Symphyla z.B. Scutigerella immaculata.
   Aus der Ordnung der Thysanura z.B. Lepisma saccharina.
   Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
   Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp..
   Aus der Ordnung der Mallophaga z.B. Trichodectes spp., Damalinea spp.
   Aus der Ordnung der Thysanoptera z.B. Frankliniella occidentalis, Hercinothrips femoralis, Thrips palmi, Thrips tabaci.
   Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium comi, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrelta, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Liriomyza spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
   Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen Wirkstoffe zeichnen sich durch eine hohe insektizide und akarizide Wirksamkeit nach Blatt- und Bodenanwendung aus.

Sie lassen sich mit besonders gutem Erfolg zur Bekämpfung von pflanzenschädigenden Insekten einzetzen, wie beispielsweise gegen die Larven des Meerrettichblattkäfers (Phaedon cochleariae), gegen die Larven der grünen Reiszikade (Nephotettix cincticeps) und gegen die Larven der grünen Pfirsichblattlaus (Myzus persicae).

Die erfindungsgemäßen Wirkstoffe besitzen auch eine herbizide Wirksamkeit und können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die zur Unkrautbekämpfung notwendigen Dosierungen der erfindungsgemäßen Wirkstoffe liegen zwischen 0,001 und 10 kg/ha, vorzugsweise zwischen 0,005 und 5 kg/ha.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondem erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Außerdem weisen die erfindungsgemäßen 3-Phenyl-pyrone der Formel (I) auch eine starke fungizide Wirkung auf und können zur Bekämpfung von unerwünschten pilzlichen Mikroorganismen im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Beispielhaft aber nicht begrenzend seien einige Erreger von pilzlichen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Stoffe mit besonders gutem Erfolg zur Bekämpfung von Krankheiten im Wein-, Obst- und Gemüseanbau, wie beispielsweise gegen Plasmopara, einsetzen. Sie zeigen außerdem eine gute Wirkung gegen Pyricularia oryzae an Reis und besitzen sowohl in -vitro als auch in -vivo eine breite fungizide Wirkung.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 % und daneben bevorzugt Streckmittel und/oder oberflächenaktive Mittel.

Die erfindungsgemäßen Wirkstoffe können in handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan, Kasugamycin, Kupfer-Zubereitungen, wie: Kupferhydroxid, Kupfemaphthenat, Kupferoxychlorid, Kupfersulfat, Kupferoxid, Oxin-Kupfer und Bordeaux-Mischung, Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol, Validamycin A, Vinclozolin,
   Zineb, Ziram.
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, AC 303 630, Acephat, Acrinathrin, Alanycarb, Aldicarb, Alphamethrin, Amifraz, Avermectin, AZ 60541, Azadirachtin, Azinphos A, Azinphos M, Azocyclotin,
   Bacillus thuringiensis, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivermectin, Lambda-cyhalothrin, Lufenuron,
   Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram,
   Omethoat, Oxamyl, Oxydemethon M, Oxydeprofos,
   Parathion A, Parathion M, Permethrin, Phenthoat, Phorat, Phosalon, Phosmet, Phosphamidon, Phoxim, Pirimicarb, Pirimiphos M, Pirimiphos A, Profenofos, Promecarb, Propaphos, Propoxur, Prothiofos, Prothoat, Pymetrozin, Pyrachlophos, Pyridaphenthion, Pyresmethrin, Pyrethrum, Pyridaben, Pyrimidifen, Pyriproxifen, Quinalphos,
   RH 5992,
   Salithion, Sebufos, Silafluofen, Sulfotep, Sulprofos,
   Tebufenozid, Tebufenpyrad, Tebupirimiphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, YI 5301/5302, Zetamethrin.
**Herbizide:**
   beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuronmethyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp.,
Aus der Ordnung der Heteropterida z.B. Cimex spp., Triatoma spp., Rhodnius spp., Panstrongylus spp..
Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Stemostoma spp., Varroa spp..
Aus der Ordnung der Actinedida (Prostigmata) und Acaridida (Astigmata) z.B. Acarapis spp., Cheyletiella spp., Omithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpudems sowie mit Hilfe von wirkstoffhaltigen Formkörpem, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen der Formel (I) eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
Hautflügler wie
   Sirex juvencus, Urocerus gigas, Urocerus gigas taignus, Urocerus augur.
Termiten wie
   Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie
   Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz und Holzverarbeitungsprodukte und Anstrichmittel.

Ganz besonders bevorzugt handelt es sich bei dem vor Insektenbefall zu schützenden Material um Holz und Holzverarbeitungsprodukte.

Unter Holz und Holzverarbeitungsprodukten, welche durch das erfindungsgemäße Mittel bzw. dieses enthaltende Mischungen geschützt werden kann, ist beispielhaft zu verstehen: Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln.

Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/ oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise - Monochlomaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, daß das Lösungsmittelgemisch ebenfalls eine Verdunstungszahl über 35 und einen Flammpunkt oberhalb 30°C, vorzugsweise oberhalb 45°C, aufweist und daß das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner seien Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron und Triflumuron, sowie Fungizide wie Epoxyconazole, Hexaconazole, Azaconazole, Propiconazole, Tebuconazole, Cyproconazole, Metconazole, Imazalil, Dichlofluanid, Tolylfluanid, 3-Iod-2-propinyl-butylcarbamat, N-Octyl-isothiazolin-3-on und 4,5-Dichlor-N-octylisothiazolin-3-on genannt.

Die Herstellung und die Verwendung der erfindungsgemäßen Wirkstoffe geht aus den nachfolgenden Beispielen hervor.

### Herstellungsbeispiele

### Beispiel 1

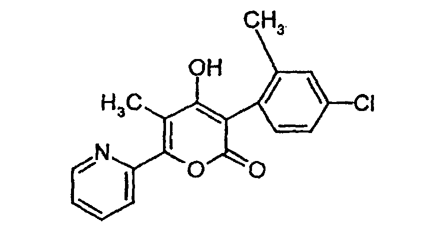

In ein Gemisch aus 4,6 g (20 mmol) (Chlorcarbonyl)-2-(2-methyl-4-chlor-phenyl)-keten und 40 ml wasserfreiem Toluol werden bei Raumtemperatur unter Rühren 2,7 g (20 mmol) Ethyl-(pyrid-2-yl)-keton gegeben. Das entstehende Gemisch wird 8 Stunden unter Rückfluß erhitzt. Nach dem Abkühlen auf Raumtemperatur wird der anfallende Niederschlag abgesaugt und zweimal mit Cyclohexan gewaschen. Man erhält auf diese Weise 3,4 g (51 % der Theorie) an 3-(2-Methyl-4-chlor-phenyl)-4-hydroxy-5-methyl-6-(pyrid-2-yl)-pyron in Form einer Festsubstanz vom Schmelzpunkt 133-135°C.

Nach der zuvor angegebenen Methode werden auch die in der folgenden Tabelle 4 aufgeführten 3-Phenyl-pyrone der Formel (I) hergestellt.

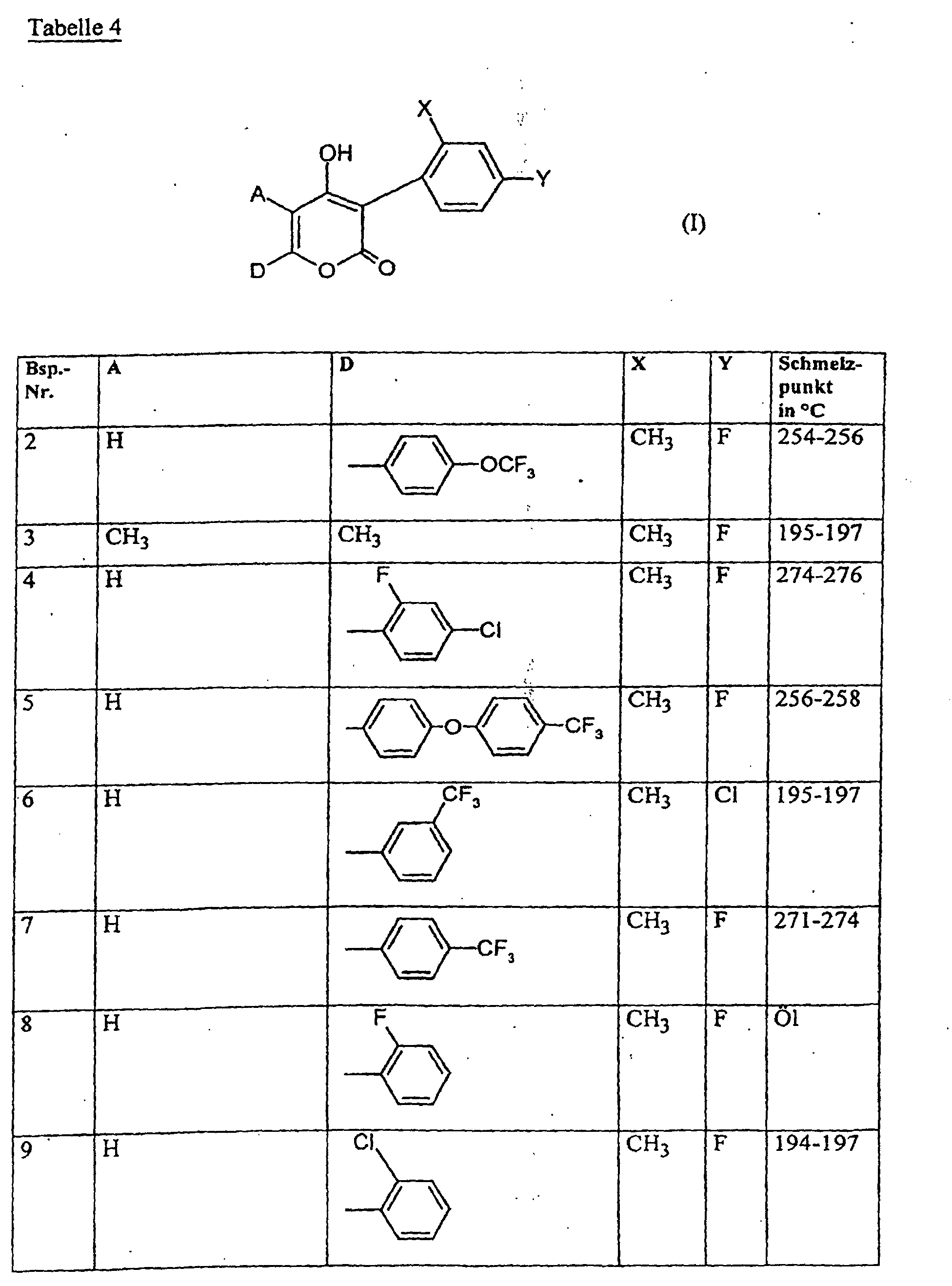

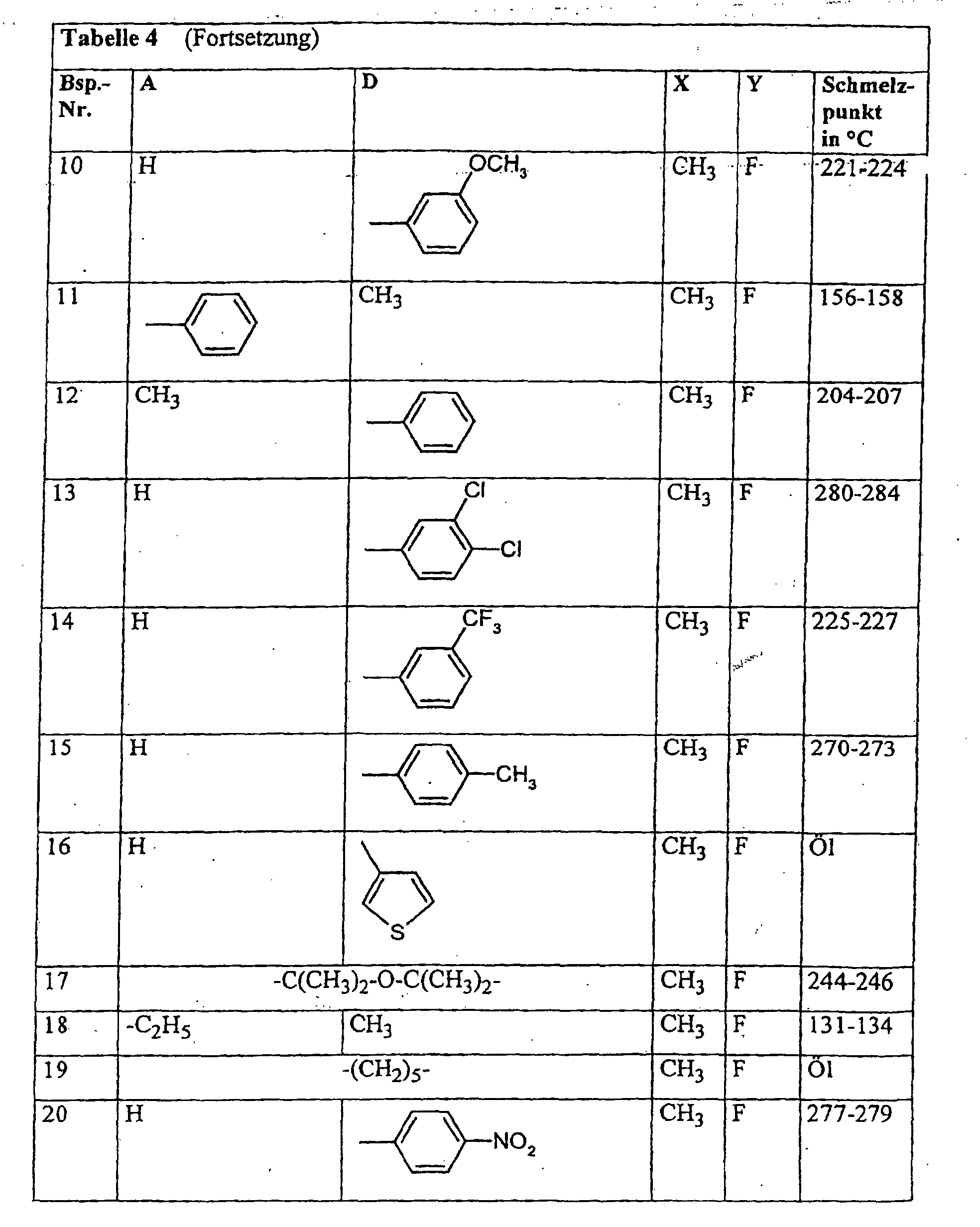

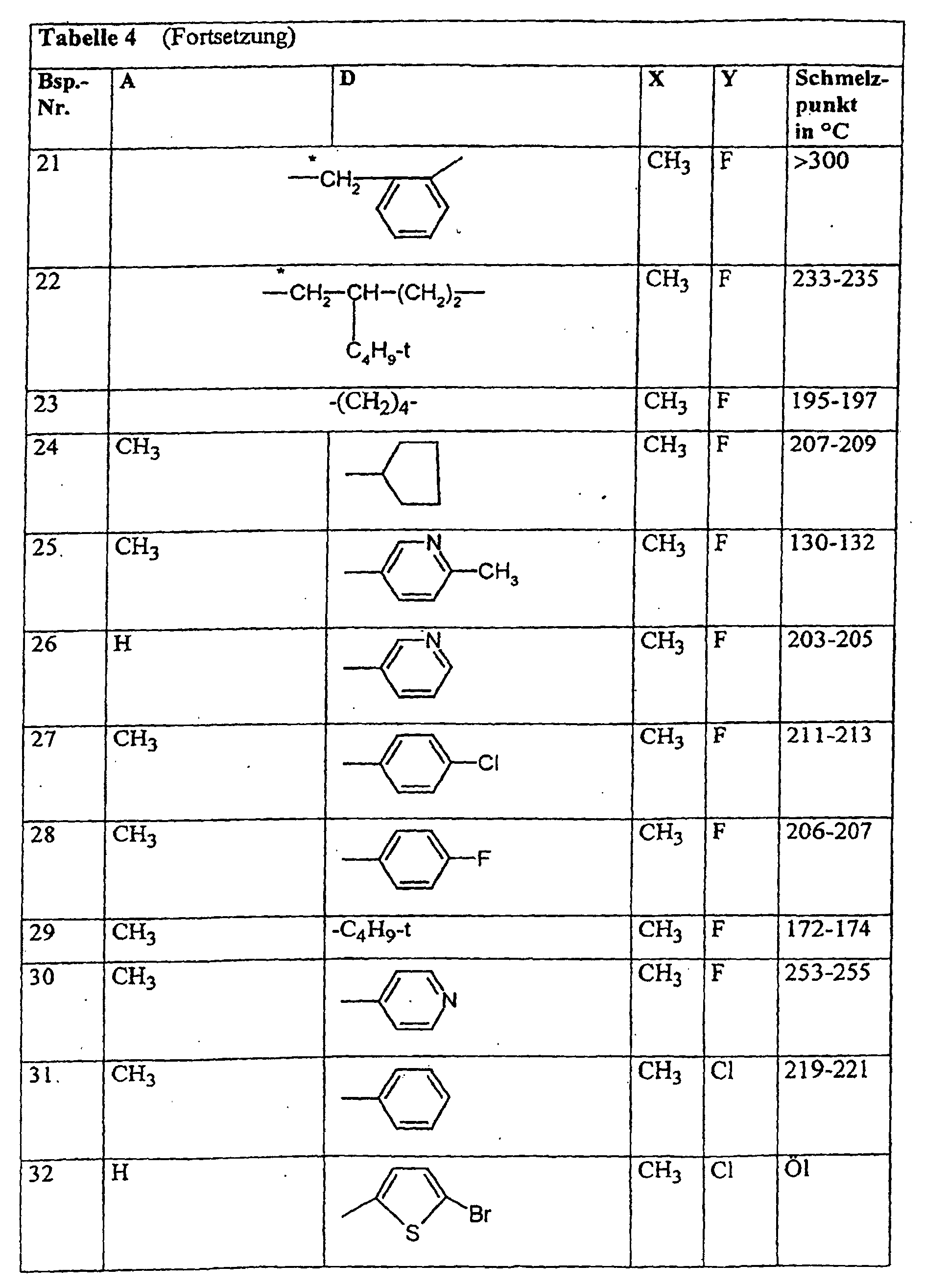

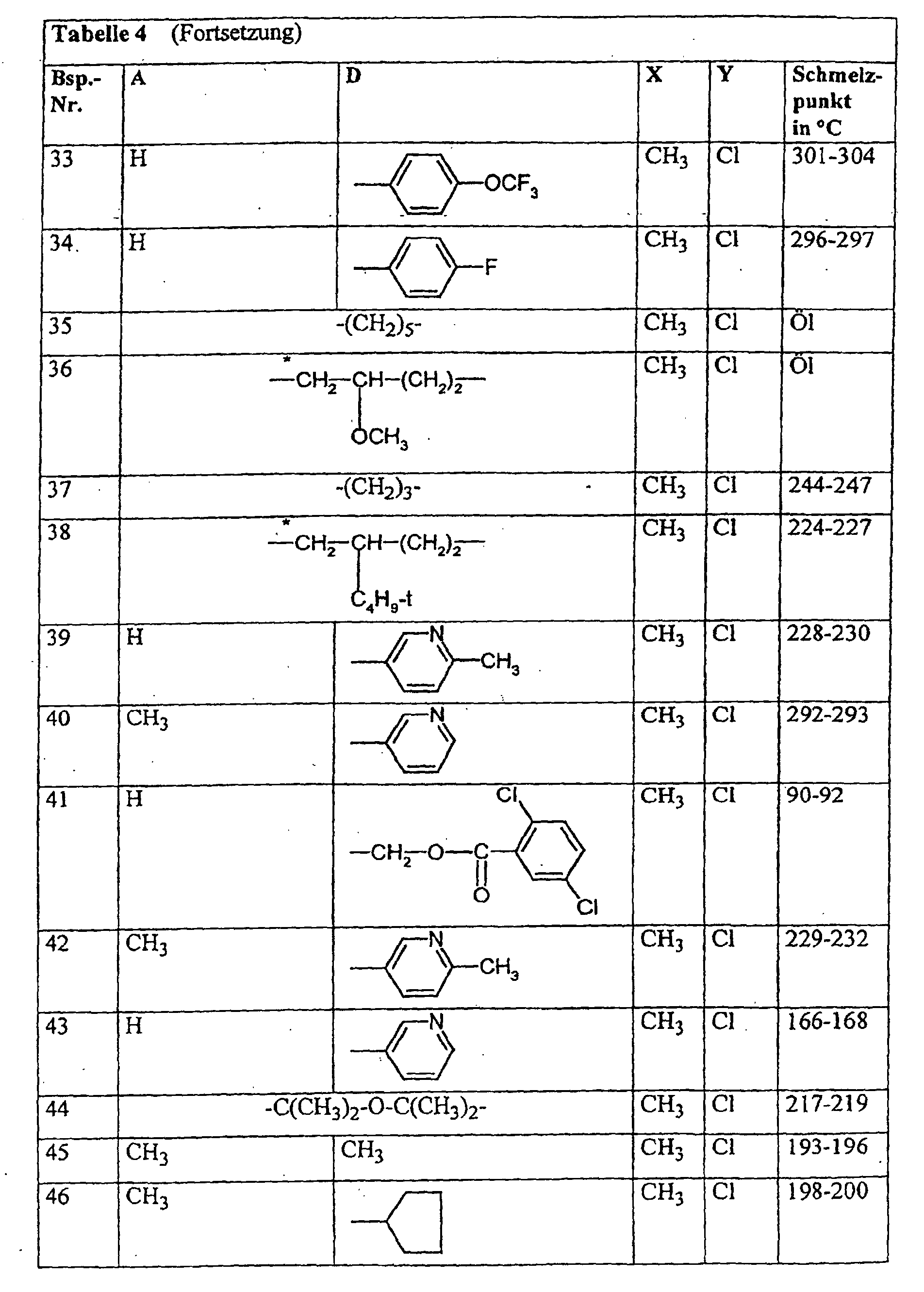

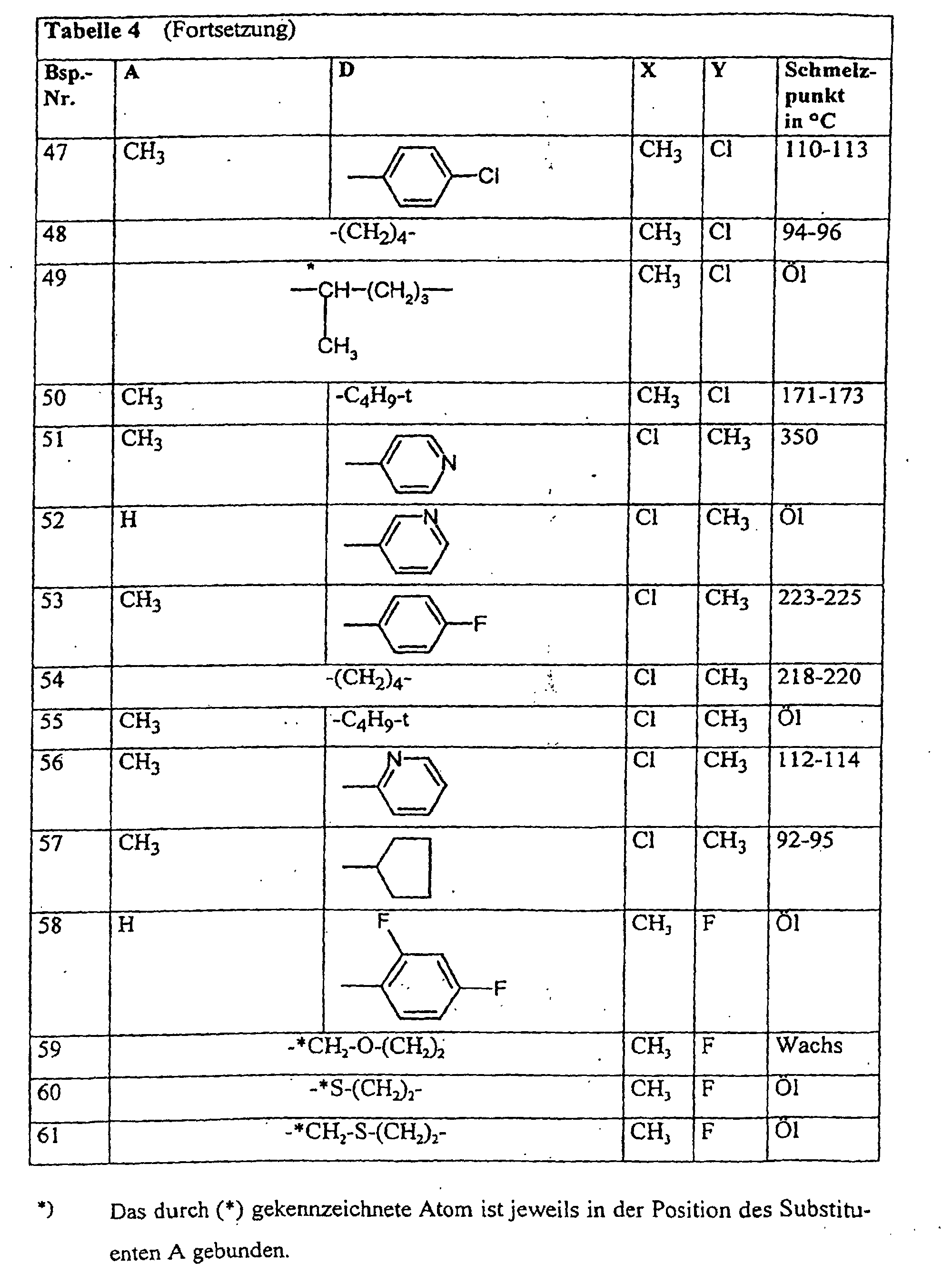

### Verwendungsbeispiele

### Beispiel A

### Myzus-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test zeigen die in den Beispielen 1, 42, 46, 56 und 57 offenbarten erfindungsgemäßen Stoffe bei einer Wirkstoffkonzentration von 0,1 % in der Wirkstoffzubereitung einen Wirkungsgrad von über 90 Prozent.

### Beispiel B

### Nephotettix-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Reiskeimlinge (Oryza sativa) werden durch Tauchen in die Wirkstoffzubereitung der gewünschten Konzentration behandelt und mit der Grünen Reiszikade (Nephotettix cincticeps) besetzt, solange die Blätter noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

In diesem Test zeigen die in den Beispielen 28, 44, 45, 55 und 56 offenbarten erfindungsgemäßen Stoffe bei einer Wirkstoffkonzentration von 0,1 % in der Wirkstoffzubereitung einen Wirkungsgrad von 100 Prozent.

### Beispiel C

### Phaedon-Larven-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Larven Meerrettichkäfers (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käferlarven abgetötet wurden.

In diesem Test zeigen die in den Beispielen 1 und 56 offenbarten erfindungsgemäßen Stoffe bei einer Wirkstoffkonzentration von 0,1 % in der Wirkstoffzubereitung einen Wirkungsgrad von 100 Prozent.

### Beispiel D

### Plutella-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella xylostella) besetzt, solange die Blätter noch feucht sind.

Nach der gewünschten Zeit wird die Abtötung in % bestimmt. Dabei bedeutet 100 %, daß alle Raupenlarven abgetötet wurden; 0 % bedeutet, daß keine Raupen abgetötet wurden.

In diesem Test zeigen die in den Beispielen 24, 45, 46 und 50 offenbarten erfindungsgemäßen Stoffe bei einer Wirkstoffkonzentration von 0,1 % in der Wirkstoffzubereitung einen Wirkungsgrad von 100 Prozent.

### Beispiel E

### Spodoptera frugiperda-Test

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Köhlblätter (Brassica oleracea) werden durch Tauchen in die Wirkstoffzubereitung der gewünschten Konzentration behandelt und mit Raupen des Heerwurms (Spodoptera frugiperda) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test zeigen die in den Beispielen 44 und 56 offenbarten erfindungsgemäßen Stoffe bei einer Wirkstoffkonzentration von 0,1 % in der Wirkstoffzubereitung einen Wirkungsgrad von 100 Prozent.

### Beispiel F

### Tetranychus-Test (OP-resistent/Tauchbehandlung)

| | |
|---|---|
| Lösungsmittel | 7 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt Dabei bedeutet 100 %, daß alle Spinnmilben abgetötet würden; 0 % bedeutet, daß keine Spinnmilben abgetötet wurden.

In diesem Test zeigen die in den Beispielen 3, 29, 44, 46 und 50 offenbarten erfindungsgemäßen Stoffe bei einer Wirkstoffkonzentration von 0,1 % in der Wirkstoffzubereitung einen Wirkungsgrad von über 90 Prozent.

### Beispiel G

### Grenzkonzentrations-Test / Wurzelsystemische Wirkung

| | |
|---|---|
| Testinsekt | **Aphis fabae** |
| Lösungsmittel | 4 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in 250 ml Töpfe und bepflanzt diese mit vorgekeimten Ackerbohnen. Der Wirkstoff kann so von den Pflanzenwurzeln aus dem Boden aufgenommen und in die Blätter transportiert werden.
Für den Nachweis des wurzelsystemischen Effektes werden nach 7 Tagen die Blätter mit den obengenannten Testtieren besetzt. Nach weiteren 7 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0%, wenn noch genau so viele Testinsekten leben wie bei der unbehandelten Kontrolle.

In diesem Test zeigt der in Beispiel 1 offenbarte erfindungsgemäße Wirkstoff bei einer Wirkstoffkonzentration von 1,25 ppm im Boden einen Wirkungsgrad von 100 Prozent.

### Beispiel H

### Grenzkonzentrations-Test / Wurzelsystemische Wirkung

| | |
|---|---|
| Testinsekt | **Myzus persicae** |
| Lösungsmittel | 4 Gewichtsteile Dimethylformamid |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Mengen Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffes in der Zubereitung praktisch keine Rolle, entscheidend ist allein die Wirkstoffgewichtsmenge pro Volumeneinheit Boden, welche in ppm (mg/ml) angegeben wird. Man füllt den behandelten Boden in 250 ml Töpfe und bepflanzt diese mit vorgekeimten Ackerbohnen. Der Wirkstoff kann so von den Pflanzenwurzeln aus dem Boden aufgenommen und in die Blätter transportiert werden.

Für den Nachweis des wurzelsystemischen Effektes werden nach 7 Tagen die Blätter mit den obengenannten Testtieren besetzt. Nach weiteren 7 Tagen erfolgt die Auswertung durch Zählen oder Schätzen der toten Tiere. Aus den Abtötungszahlen wird die wurzelsysteinische Wirkung des Wirkstoffs abgeleitet. Sie ist 100 %, wenn alle Testtiere abgetötet sind und 0%, wenn noch genau so viele Testinsekten leben wie bei der unbehandelten Kontrolle.

In diesem Test zeigt der in Beispiel 1 offenbarte erfindungsgemäße Wirkstoff bei einer Wirkstoffkonzentration von 1,25 ppm im Boden einen Wirkungsgrad von 100 Prozent.

### Beispiel J

### Plasmopara-Test (Rebe)/protektiv

| | |
|---|---|
| Lösungsmittel | 47 Gewichtsteile Aceton |
| Emulgator | 3 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Plasmopara viticola inokuliert und verbleiben dann 1 Tag in einer Inkubationskabine bei ca. 20°C und 100 % relativer Luftfeuchtigkeit. Anschließend werden die Pflanzen 5 Tage im Gewächshaus bei ca. 21°C und ca. 90 % Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

In diesem Test zeigt der in Beispiel 57 offenbarte erfindungsgemäße Wirkstoff bei einer Aufwandmenge von 100 g/ha einen Wirkungsgrad von über 80 Prozent.

## Patentansprüche

1. 3-Phenyl-pyrone der Formel 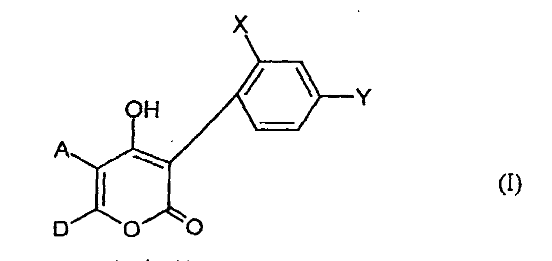 in welcher
X für Alkyl mit 1 bis 6 Kohlenstoffatomen steht und
Y für Fluor, Chlor oder Brom steht
oder
X für Fluor, Chlor oder Brom steht und
Y für Alkyl mit 1 bis 6 Kohlenstoffatomen steht,
A für Wasserstoff, Alkyl mit 1 bis 12 Kohlenstoffatomen oder für Phenyl steht, das einfach bis dreifach, gleichartig oder verschieden substituiert sein kann durch Halogen, C₁-C₆-Alkyl, C₁-C₆-Halogenalkyl, C₁-C₆-Alkoxy, C₁-C₆-Halogenalkoxy, Cyano und/oder Nitro,
D für Wasserstoff, Alkyl mit 1 bis 12 Kohlenstoffatomen, für gegebenenfalls durch Halogen, Alkyl mit 1 bis 4 Kohlenstoffatomen, Alkoxy mit 1 bis 4 Kohlenstoffatomen und/oder Halogenalkyl mit 1 bis 4 Kohlenstoffatomen substituiertes Cycloalkyl mit 3 bis 8 Kohlenstoffatomen steht,
oder für Phenyl steht, das einfach bis dreifach, gleichartig oder verschieden substituiert sein kann durch Halogen, C₁-C₆-Alkyl, C₁-C₆-Halogenalkyl, C₁-C₆-Alkoxy, C₁-C₆-Halogenalkoxy, Cyano, Nitro, Phenyl und/oder Phenoxy, wobei die beiden letztgenannten Reste ihrerseits einfach oder zweifach substituiert sein können durch Halogen, C₁-C₄-Alkyl, C₁-C₄-Alkoxy, C₁-C₄-Halogenalkyl und/oder C₁-C₄-Halogenalkoxy,
oder für fünf- oder sechsgliedriges Heteroaryl mit 1 oder 2 Heteroatomen aus der Reihe Sauerstoff, Schwefel und Stickstoff steht, wobei diese Reste einfach oder zweifach substituiert sein können durch Halogen, C₁-C₆-Alkyl, C₁-C₆-Halogenalkyl, C₁-C₆-Alkoxy, C₁-C₆-Halogenalkoxy, Cyano und/oder Nitro,
oder für einen Rest der Formel 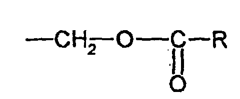 steht,
worin
R für Phenyl steht, das einfach bis dreifach, gleichartig oder verschieden substituiert sein kann durch Halogen, C₁-C₆-Alkyl, C₁-C₆-Halogenalkyl, C₁-C₆-Alkoxy, C₁-C₆-Halogenalkoxy, Cyano und/oder Nitro,
A und D außerdem auch gemeinsam für eine C₃-C₆-Alkandiyl-, C₃-C₆-Alkendiyl- oder C₄-C₆-Alkandiendiylgruppe stehen, worin jeweils gegebenenfalls eine Methylengruppe durch Sauerstoff oder Schwefel ersetzt ist, und welche jeweils gegebenenfalls substituiert sind durch Halogen, C₁-C₆-Alkyl, C₁-C₆-Halogenalkyl, C₁-C₆-Alkoxy, C₁-C₆-Halogenalkoxy, oder durch eine weitere, einen ankondensierten Ring bildende C₃-C₆-Alkandiyl-, C₃-C₆-Alkendiyl- oder C₄-C₆-Alkandiendiylgruppe, in welchen gegebenenfalls jeweils eine Methylengruppe durch Sauerstoff oder Schwefel ersetzt ist und welche gegebenenfalls durch C₁-C₆-Alkyl substituiert sind,
wobei die Verbindungen der Formeln 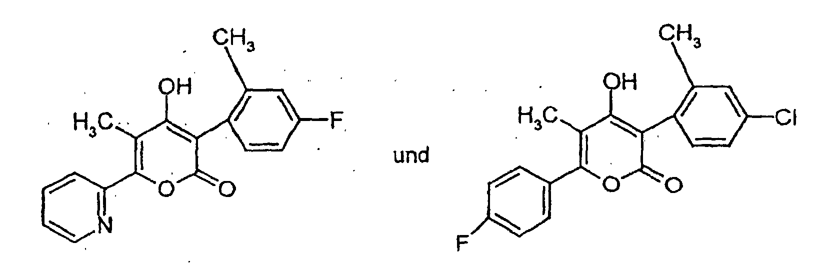 ausgenommen sind.

2. Verbindungen der Formel (I) gemäß Anspruch 1, in welcher
X für Alkyl mit 1 bis 4 Kohlenstoffatomen steht und
Y für Fluor, Chlor oder Brom steht
oder
X für Chlor oder Brom steht und
Y für Alkyl mit 1 bis 4 Kohlenstoffatomen steht,
A für Wasserstoff, Alkyl mit 1 bis 8 Kohlenstoffatomen oder für Phenyl steht, das einfach bis dreifach, gleichartig oder verschieden substituiert sein kann durch Fluor, Chlor, Brom, C₁-C₄-Alkyl, C₁-C₄-Halogenalkyl, C₁-C₄-Alkoxy, C₁-C₄-Halogenalkoxy, Cyano und/oder Nitro,
D für Wasserstoff, Alkyl mit 1 bis 10 Kohlenstoffatomen, für gegebenenfalls einfach bis dreifach, gleichartig oder verschieden durch Fluor, Chlor, C₁-C₃-Alkyl, C₁-C₂-Alkoxy, C₁-C₂-Halogenalkyl substituiertes Cycloalkyl mit 3 bis 7 Kohlenstoffatomen steht,
oder für Phenyl steht, das einfach bis dreifach, gleichartig oder verschieden substituiert sein kann durch Fluor, Chlor, Brom, C₁-C₄-Alkyl, C₁-C₄-Halogenalkyl, C₁-C₄-Alkoxy, C₁-C₄-Halogenalkoxy, Cyano, Nitro, Phenyl und/oder Phenoxy, wobei die beiden letztgenannten Reste ihrerseits einfach oder zweifach substituiert sein können durch Fluor, Chlor, Brom, Methyl, Ethyl, Methoxy, Trifluormethyl, Trifluormethoxy und/oder Difluormethoxy,
oder für Furanyl, Pyridyl, Imidazolyl, Triazolyl, Pyrazolyl, Pyrimidinyl, Thiazolyl oder Thienyl steht, wobei diese Reste einfach oder zweifach substituiert sein können durch Fluor, Chlor, Brom, C₁-C₄-Alkyl, C₁-C₄-Halogenalkyl, C₁-C₄-Alkoxy, C₁-C₄-Halogenalkoxy, Cyano und/oder Nitro,
oder
D für einen Rest der Formel 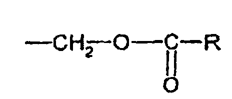 steht,
worin
R für Phenyl steht, das einfach bis dreifach, gleichartig oder verschieden substituiert sein kann durch Fluor, Chlor, Brom, C₁-C₄-Alkyl, C₁-C₄-Halogenalkyl, C₁-C₄-Alkoxy, C₁-C₄-Halogenalkoxy, Cyano und/oder Nitro,
A und D außerdem gemeinsam für eine C₃-C₅-Alkandiyl oder C₃-C₅-Alkendiylgruppe stehen, worin jeweils gegebenenfalls ein Kohlenstoffatom durch Sauerstoff oder Schwefel ersetzt ist, und welche gegebenenfalls substituiert sind durch Fluor, Chlor, Brom, C₁-C₄-Alkyl, C₁-C₄-Alkoxy, C₁-C₄-Halogenalkyl und/oder C₁-C₆-Halogenalkoxy, oder durch eine weitere, einen ankondensierten Ring bildende C₄-Alkandiendiylgruppe, die durch C₁-C₄-Alkyl substituiert sein kann,
wobei die Verbindungen der Formeln 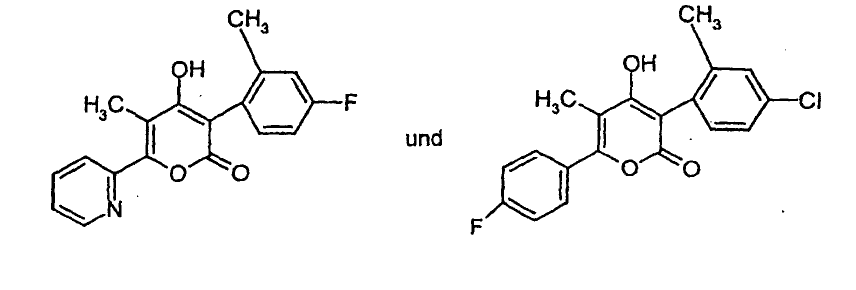 ausgenommen sind.

3. Verbindungen der Formel (I) gemäß Anspruch 1, in welcher
X für Methyl oder Ethyl steht und
Y für Fluor oder Chlor steht
oder
X für Chlor oder Brom steht und
Y für Methyl oder Ethyl steht,
A für Wasserstoff, Alkyl mit 1 bis 6 Kohlenstoffatomen oder für Phenyl steht, das einfach oder zweifach, gleichartig oder verschieden substituiert sein kann durch Fluor, Chlor, Methyl, Ethyl, n-Propyl, Isopropyl, Trifluormethyl, Methoxy, Ethoxy, Trifluormethoxy, Cyano und/oder Nitro,
D für Wasserstoff, Alkyl mit 1 bis 8 Kohlenstoffatomen steht,
oder für gegebenenfalls einfach oder zweifach, gleichartig oder verschieden durch Fluor, Chlor, Methyl, Ethyl, Methoxy, Ethoxy und/oder Trifluormethyl substituiertes Cycloalkyl mit 3 bis 7 Kohlenstoffatomen steht,
oder für Phenyl steht, das einfach oder zweifach, gleichartig oder verschieden substituiert sein kann durch Fluor, Chlor, Brom, Methyl, Ethyl, n-Propyl, Isopropyl, n-Butyl, sek.-Butyl, iso-Butyl, tert.-Butyl, Trifluormethyl, Methoxy, Ethoxy, Trifluormethoxy, Cyano, Nitro, Phenyl und/oder Phenoxy, wobei die beiden letztgenannten Reste ihrerseits einfach oder zweifach substituiert sein können durch Fluor, Chlor, Methyl, Methoxy, Trifluormethyl und/oder Trifluormethoxy,
oder für Furanyl, Pyridyl oder Thienyl steht, wobei diese Reste einfach oder zweifach substituiert sein können durch Fluor, Chlor, Brom, Methyl, Ethyl, n-Propyl, Isopropyl, Trifluormethyl, Trifluormethoxy, Cyano und/oder Nitro,
oder
D für einen Rest der Formel 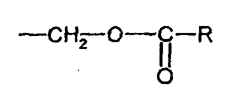 steht,
worin
R für Phenyl steht, das einfach oder zweifach, gleichartig oder verschieden substituiert sein kann durch Fluor, Chlor, Methyl, Ethyl, Trifluormethyl, Methoxy, Trifluormethoxy, Cyano und/oder Nitro,
A und D außerdem gemeinsam für eine C₃-C₅-Alkandiyl oder C₃-C₅-Alkendiylgruppe, worin jeweils gegebenenfalls eine Methylengruppe durch Sauerstoff oder Schwefel ersetzt ist, und welche gegebenenfalls einfach bis vierfach, gleichartig oder verschieden substituiert ist durch Fluor, Chlor, Brom, Methyl, Ethyl, n-Propyl, Isopropyl, n-Butyl, iso-Butyl, sek.-Butyl, tert.-Butyl, Methoxy, Trifluormethyl und/oder Trifluormethoxy, oder durch eine weitere, einen ankondensierten Ring bildende Butadiendiylgruppe stehen, die einfach oder zweifach durch Methyl substituiert sein kann,
wobei die Verbindungen der Formeln 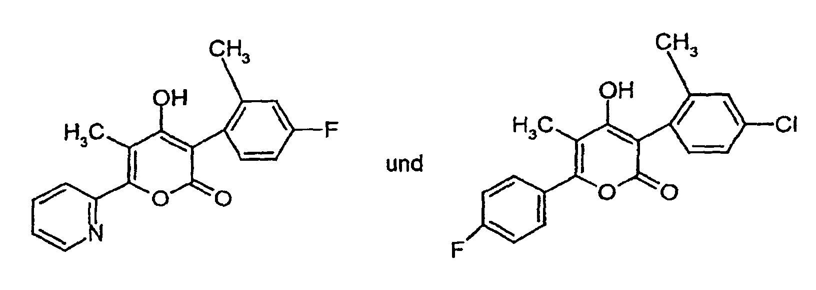 ausgenommen sind.

4. Verfahren zur Herstellung von 3-Phenyl-pyronen der Formel (I) gemäß Anspruch 1, **dadurch gekennzeichnet, daß** man
α) entweder Carbonyl-Verbindungen der Formel 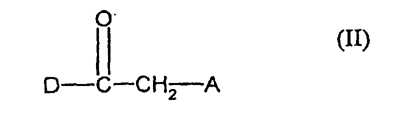
in welcher
A und D die oben angegebenen Bedeutungen haben,
oder
β) Silylether der Formel 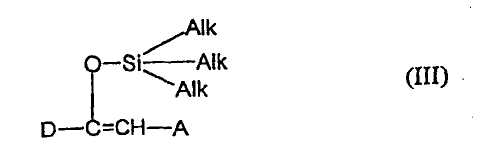 in welcher
A und D die oben angegebenen Bedeutungen haben und
Alk für Alkyl mit 1 bis 4 Kohlenstoffatomen steht,
jeweils mit Keten-Derivaten der Formel. 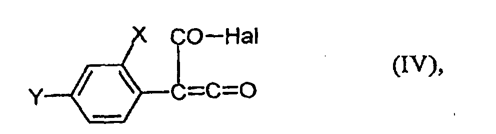 in welcher
X und Y die oben angegebenen Bedeutungen haben und
Hal für Chlor oder Brom steht,
gegebenenfalls in Gegenwart eines Verdünnungsmittels und gegebenenfalls in Gegenwart eines Säureakzeptors umsetzt.

5. Pestizide, fungizide und herbizide Mittel, **gekennzeichnet durch** einen Gehalt an mindestens einem 3-Phenyl-pyron der Formel (I) gemäß Anspruch 1 neben Streckmitteln und/oder oberflächenaktiven Stoffen.

6. Verwendung von 3-Phenyl-pyronen der Formel (I) gemäß Anspruch 1 als Pestizide, Fungizide und Herbizide.

7. Verfahren zur Bekämpfung von tierischen Schädlingen, **dadurch gekennzeichnet, daß** man 3-Phenyl-pyrone der Formel (I) gemäß Anspruch 1 auf die Schädlinge und/oder deren Lebensraum ausbringt.

8. Verfahren zur Bekämpfung von Pilzen, **dadurch gekennzeichnet, daß** man 3-Phenyl-pyrone der Formel (I) gemäß Anspruch 1 auf die Pilze und/oder deren Lebensraum ausbringt.

9. Verfahren zur Bekämpfung von Unkräutern, **dadurch gekennzeichnet, daß** man 3-Phenyl-pyrone der Formel (I) gemäß Anspruch 1 auf die Unkräuter und/oder deren Lebensraum ausbringt.

10. Verfahren zur Herstellung von pestiziden, fungiziden und herbiziden Mitteln, **dadurch gekennzeichnet, daß** man 3-Phenyl-pyrone der Formel (I) gemäß Anspruch 1 mit Streckmitteln und/oder oberflächenaktiven Stoffen vermischt.

## Claims

1. 3-phenyl-pyrones of the formula 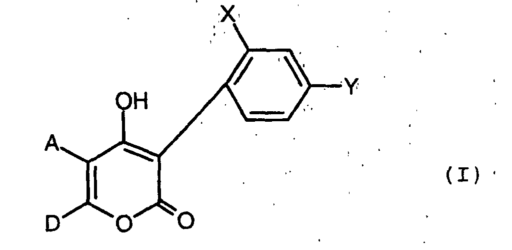 in which
X represents alkyl having 1 to 6 carbon atoms and
Y represents fluorine, chlorine or bromine
or
X represents fluorine, chlorine or bromine and
Y represents alkyl having 1 to 6 carbon atoms,
A represents hydrogen, alkyl having 1 to 12 carbon atoms or represents phenyl which may be mono- to trisubstituted by identical or different substituents selected from the group consisting of halogen, C₁-C₆-alkyl, C₁-C₆-halogenoalkyl, C₁-C₆-alkoxy, C₁-C₆-halogenoalkoxy, cyano and nitro,
D represents hydrogen, alkyl having 1 to 12 carbon atoms, represents cycloalkyl having 3 to 8 carbon atoms which is optionally substituted by halogen, alkyl having 1 to 4 carbon atoms, alkoxy having 1 to 4 carbon atoms and/or halogenoalkyl having 1 to 4 carbon atoms,
or represents phenyl which may be mono- to trisubstituted by identical or different substituents selected from the group consisting of halogen, C₁-C₆-alkyl, C₁-C₆-halogenoalkyl, C₁-C₆-alkoxy, C₁-C₆-halogenoalkoxy, cyano, nitro, phenyl and/or phenoxy, where the two last mentioned radicals for their part may be mono- or disubstituted by halogen, C₁-C₄-alkyl, C₁-C₄-alkoxy, C₁-C₄-halogenoalkyl and/or C₁-C₄-halogenoalkoxy,
or represents 5- or 6-membered heteroaryl having 1 or 2 heteroatoms selected from the group consisting of oxygen, sulphur and nitrogen, where these radicals may be mono- or disubstituted by halogen, C₁-C₆-alkyl, C₁-C₆-halogenoalkyl, C₁-C₆-alkoxy, C₁-C₆-halogenoalkoxy, cyano and/or nitro,
or represents a radical of the formula 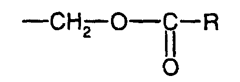 in which
R represents phenyl which may be mono- to trisubstituted by identical or different substituents selected from the group consisting of halogen, C₁-C₆-alkyl, C₁-C₆-halogenoalkyl, C₁-C₆-alkoxy, C₁-C₆-halogenoalkoxy, cyano and/or nitro,
A and D together furthermore also represent a C₃-C₆-alkanediyl, C₃-C₆-alkenediyl or C₄-C₆-alkanedienediyl group in which in each case optionally one methylene group is replaced by oxygen or sulphur, and which are in each case optionally substituted by halogen, C₁-C₆-alkyl, C₁-C₆-halogenoalkyl, C₁-C₆-alkoxy, C₁-C₆-halogenoalkoxy, or by a further C₃-C₆-alkanediyl-, C₃-C₆-alkenediyl- or C₄-C₆-alkanedienediyl group which forms a fused-on ring and in which in each case optionally one methylene group is replaced by oxygen or sulphur and which are optionally substituted by C₁-C₆-alkyl,
except for the compounds of the formulae

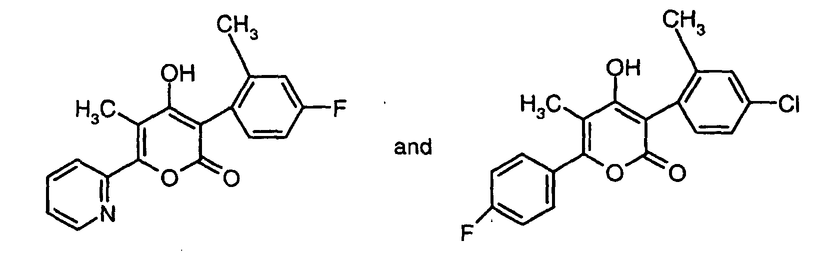

2. Compounds of the formula (I) according to Claim 1 in which
X represents alkyl having 1 to 4 carbon atoms and
Y represents fluorine, chlorine or bromine
or
X represents chlorine or bromine and
Y represents alkyl having 1 to 4 carbon atoms,
A represents hydrogen, alkyl having 1 to 8 carbon atoms or represents phenyl which may be mono- to trisubstituted by identical or different substituents selected from the group consisting of fluorine, chlorine, bromine, C₁-C₄-alkyl, C₁-C₄-halogenoalkyl, C₁-C₄-alkoxy, C₁-C₄-halogenoalkoxy, cyano and nitro,
D represents hydrogen, alkyl having 1 to 10 carbon atoms, represents cycloalkyl having 3 to 7 carbon atoms which is optionally mono- to trisubstituted by identical or different substituents selected from the group consisting of fluorine, chlorine, C₁-C₃-alkyl, C₁-C₂-alkoxy, C₁-C₂-halogenoalkyl,
or represents phenyl which may be mono- to trisubstituted by identical or different substituents selected from the group consisting of fluorine, chlorine, bromine, C₁-C₄-alkyl, C₁-C₄-halogenoalkyl, C₁-C₄-alkoxy, C₁-C₄-halogenoalkoxy, cyano, nitro, phenyl and/or phenoxy, where the two last mentioned radicals for their part may be mono- or disubstituted by fluorine, chlorine, bromine, methyl, ethyl, methoxy, trifluoromethyl, trifluoromethoxy and/or difluoromethoxy,
or represents furanyl, pyridyl, imidazolyl, triazolyl, pyrazolyl, pyrimidinyl, thiazolyl or thienyl, where these radicals may be mono- or disubstituted by fluorine, chlorine, bromine, C₁-C₄-alkyl, C₁-C₄-halogenoalkyl, C₁-C₄-alkoxy, C₁-C₄-halogenoalkoxy, cyano and/or nitro,
or
D represents a radical of the formula 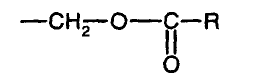 in which
R represents phenyl which may be mono- to trisubstituted by identical or different substituents selected from the group consisting of fluorine, chlorine, bromine, C₁-C₄-alkyl, C₁-C₄-halogenoalkyl, C₁-C₄-alkoxy, C₁-C₄-halogenoalkoxy, cyano and/or nitro,
A and D together furthermore represent a C₃-C₅-alkanediyl or C₃-C₅-alkenediyl group in which in each case optionally one carbon atom is replaced by oxygen or sulphur and which are optionally substituted by fluorine, chlorine, bromine, C₁-C₄-alkyl, C₁-C₄-alkoxy, C₁-C₄-halogenoalkyl and/or C₁-C₆-halogenoalkoxy, or by a further C₄-alkanedienediyl group which forms a fused-on ring and which may be substituted by C₁-C₄-alkyl,
except for the compounds of the formulae

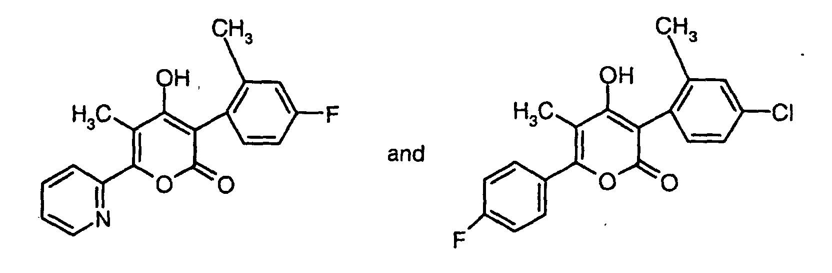

3. Compounds of the formula (I) according to Claim 1 in which
X represents methyl or ethyl and
Y represents fluorine or chlorine
or
X represents chlorine or bromine and
Y represents methyl or ethyl,
A represents hydrogen, alkyl having 1 to 6 carbon atoms or represents phenyl which may be mono- or disubstituted by identical or different substituents selected from the group consisting of fluorine, chlorine, methyl, ethyl, n-propyl, isopropyl, trifluoromethyl, methoxy, ethoxy, trifluoromethoxy, cyano and nitro,
D represents hydrogen, alkyl having 1 to 8 carbon atoms,
or represents cycloalkyl having 3 to 7 carbon atoms which is optionally mono- or disubstituted by identical or different substituents selected from the group consisting of fluorine, chlorine, methyl, ethyl, methoxy, ethoxy and/or trifluoromethyl,
or represents phenyl which may be mono- or disubstituted by identical or different substituents selected from the group consisting of fluorine, chlorine, bromine, methyl, ethyl, n-propyl, isopropyl, n-butyl, sec-butyl, iso-butyl, tert-butyl, trifluoromethyl, methoxy, ethoxy, trifluoromethoxy, cyano, nitro, phenyl a phenoxy, where the two last mentioned radicals for their part may be mono- or disubstituted by fluorine, chlorine, methyl, methoxy, trifluoromethyl and / or trifluoromethoxy,
or represents furanyl, pyridyl or thienyl, where these radicals may be mono- or disubstituted by fluorine, chlorine, bromine, methyl, ethyl, n-propyl, isopropyl, trifluoromethyl, trifluoromethoxy, cyano and/or nitro,
or
D represents a radical of the formula 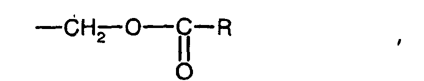 in which
R represents phenyl which may be mono- or disubstituted by identical or different substituents selected from the group consisting of fluorine, chlorine, methyl, ethyl, trifluoromethyl, methoxy, trifluoromethoxy, cyano and nitro,
A and D together furthermore represent a C₃-C₅-alkanediyl or C₃-C₅-alkenediyl group in which in each case optionally one methylene group is replaced by oxygen or sulphur and which is optionally mono- to tetrasubstituted by identical or different substituents selected from the group consisting of fluorine, chlorine, bromine, methyl, ethyl, n-propyl, isopropyl, n-butyl, iso-butyl, sec-butyl, tert-butyl, methoxy, trifluoromethyl and trifluoromethoxy, or by a further butadienediyl group which forms a fused-on ring and which may be mono- or disubstituted by methyl,
except for the compounds of the formulae

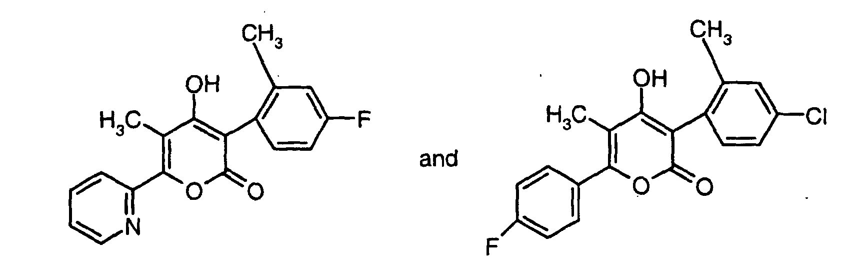

4. Process for preparing 3-phenyl-pyrones of the formula (I) according to Claim 1, **characterized in that**
α) either carbonyl compounds of the formula 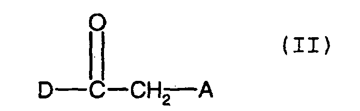
in which
A and D are each as defined above,
or
β) silyl ethers of the formula 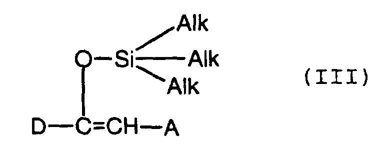 in which
A and D are each as defined above and alk represents alkyl having 1 to 4 carbon atoms
are in each case reacted with ketene derivatives of the formula 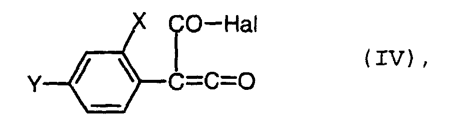 in which
X and Y are each as defined above and
Hal represents chlorine or bromine,
if appropriate in the presence of a diluent and if appropriate in the presence of an acid acceptor.

5. Pesticidal, fungicidal and herbicidal compositions, **characterized in that** they contain at least one 3-phenyl-pyrone of the formula (I) according to Claim 1, in addition to extenders and/or surfactants.

6. The use of 3-phenyl-pyrones of the formula (I) according to Claim 1 as pesticides, fungicides and herbicides.

7. Method for controlling animal pests, **characterized in that** 3-phenyl-pyrones of the formula (I) according to Claim 1 are applied to the pests and/or their habitat.

8. Method for controlling fungi, **characterized in that** 3-phenyl-pyrones of the formula (I) according to Claim 1 are applied to the fungi and/or their habitat.

9. Method for controlling weeds, **characterized in that** 3-phenyl-pyrones of the formula (I) according to Claim 1 are applied to the weeds and/or their habitat.

10. Process for preparing pesticidal, fungicidal and herbicidal compositions, **characterized in that** 3-phenyl-pyrones of the formula (I) according to Claim 1 are mixed with extenders and/or surfactants.

## Revendications

1. 3-phényl-pyrones de formule 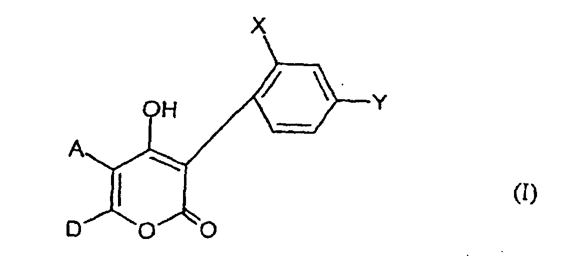 dans laquelle
X est un reste alkyle ayant 1 à 6 atomes de carbone et
Y représente le fluor, le chlore ou le brome,
ou bien
X représente le fluor, le chlore ou le brome et
Y représente un reste alkyle ayant 1 à 6 atomes de carbone,
A représente l'hydrogène, un reste alkyle ayant 1 à 12 atomes de carbone ou un reste phényle qui peut porter un à trois substituants, identiques ou différents, halogéno, alkyle en C₁ à C₆, halogénalkyle en C₁ à C₆, alkoxy en Ci à C₆, halogénalkoxy en C₁ à C₆, cyano et/ou nitro,
D un reste alkyle ayant 1 à représente l'hydrogène, un reste alkyle ayant 1 à 12 atomes de carbone, un reste cycloalkyle ayant 3 à 8 atomes de carbone portant éventuellement un substituant halogéno, alkyle ayant 1 à 4 atomes de carbone, alkoxy ayant 1 à 4 atomes de carbone et/ou halogénalkyle ayant 1 à 4 atomes de carbone,
ou bien un reste phényle qui peut porter un à trois substituants, identiques ou différents, halogéno, alkyle en C₁ à C₆, halogénalkyle en C₁ à C₆, alkoxy en C₁ à C₆, halogénalkoxy en C₁ à C₆, cyano, nitro, phényle et/ou phénoxy, les deux restes mentionnés en dernier lieu pouvant eux-mêmes porter un ou deux substituants halogéno, alkyle en C₁ à C₄, alkoxy en C₁ à C₄, halogénalkyle en C₁ à C₄ et/ou halogénalkoxy en C₁ à C₄,
ou bien un reste hétéroaryle pentagonal ou hexagonal ayant 1 ou 2 hétéroatomes de la série oxygène, soufre et azote, ces restes pouvant porter un ou deux substituants halogéno, alkyle en C₁ à C₆, halogénalkyle en C₁ à C₆, alkoxy en C₁ à C₆, halogénalkoxy en C₁ à C₆, cyano et/ou nitro,
ou un reste de formule 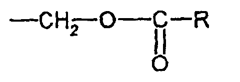 dans laquelle
R est un reste phényle qui peut porter un à trois substituants, identiques ou 'différents, halogéno, alkyle en C₁ à C₆, halogénalkyle en C₁ à C₆, alkoxy en C₁ à C₆, halogénalkoxy en C₁ à C₆, cyano et/ou nitro,
A et D forment en outre conjointement un groupe alcanediyle en C₃ à C₆, alcènediyle en C₃ à C₆ ou alcanediènediyle en C₄ à C₆, où dans chaque cas, le cas échéant, un groupe méthylène est remplacé par de l'oxygène ou du soufre, et dont chacun porte éventuellement un substituant halogéno, alkyle en C₁ à C₆, halogénalkyle en C₁ à C₆, alkoxy en C₁ à C₆, halogénalkoxy en C₁ à C₆ ou par un autre groupe alcanediyle en C₃ à C₆, alcènediyle en C₃ à C₆ ou alcanediènediyle en C₄ à C₆ formant un noyau condensé, groupes dans chacun desquels, le cas échéant, un groupe méthylène est remplacé par de l'oxygène ou du soufre et qui sont éventuellement substitués par un reste alkyle en C₁ à C₆,
les composés de formules 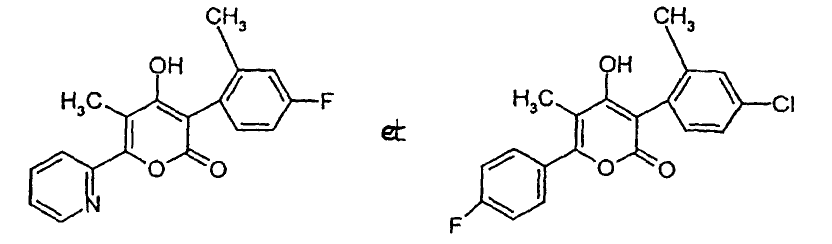 étant exclus.

2. Composés de formule (I) suivant la revendication 1, formule dans laquelle
X est un reste alkyle ayant 1 à 4 atomes de carbone et
Y représente le fluor, le chlore ou le brome,
ou bien
X est le chlore ou le brome et
Y est un reste alkyle ayant 1 à 4 atomes de carbone,
A représente l'hydrogène, un reste alkyle ayant 1 à 8 atomes de carbone ou un reste phényle qui peut porter un à trois substituants, identiques ou différents, fluoro, chloro, bromo, alkyle en C₁ à C₄, halogénalkyle en C₁ à C₄, alkoxy en C₁ à C₄, halogénalkoxy en C₁ à C₄, cyano et/ou nitro,
D représente l'hydrogène, un reste alkyle ayant 1 à 10 atomes de carbone, un reste cycloalkyle ayant 3 à 7 atomes de carbone portant éventuellement un à trois substituants, identiques ou différents, fluoro, chloro, alkyle en C₁ à C₃, alkoxy en C₁ ou C₂, halogénalkyle en C₁ ou C₂,
ou bien un reste phényle qui peut porter un à trois substituants, identiques ou différents, fluoro, chloro, bromo, alkyle en C₁ à C₄, halogénalkyle en C₁ à C₄, alkoxy en C₁ à C₄, halogénalkoxy en C₁ à C₄, cyano, nitro, phényle et/ou phénoxy, les deux restes mentionnés en dernier lieu pouvant eux-mêmes porter un ou deux substituants fluoro, chloro, bromo, méthyle, éthyle, méthoxy, trifluorométhyle, trifluorométhoxy et/ou difluorométhoxy,
ou bien un reste furannyle, pyridyle, imidazolyle, triazolyle, pyrazolyle, pyrimidinyle, thiazolyle ou thiényle, ces restes pouvant porter un ou deux substituants fluoro, chloro, bromo, alkyle en C₁ à C₄, halogénalkyle en C₁ à C₄, alkoxy en C₁ à C₄, halogénalkoxy en C₁ à C₄, cyano et/ou nitro,
ou bien
D est un reste de formule 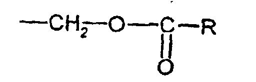 dans laquelle
R est un reste phényle qui peut porter un à trois substituants, identiques ou différents, fluoro, chloro, bromo, alkyle en C₁ à C₄, halogénalkyle en C₁ à C₄, alkoxy en C₁ à C₄, halogénalkoxy en C₁ à C₄, cyano et/ou nitro,
A et D forment en outre conjointement un groupe alcanediyle en C₃ à C₅ ou un groupe alcènediyle en C₃ à C₅, dans chacun desquels, le cas échéant, un atome de carbone est remplacé par de l'oxygène ou du soufre et qui sont éventuellement substitués par du fluor, du chlore, du brome, un radical alkyle en C₁ à C₄, alkoxy en C₁ à C₄, halogénalkyle en C₁ à C₄ et/ou halogénalkoxy en C₁ à C₄, ou par un autre groupe alcanediènediyle en C₄ formant un noyau condensé et pouvant être substitué par un reste alkyle en C₁ à C₄, les composés de formules 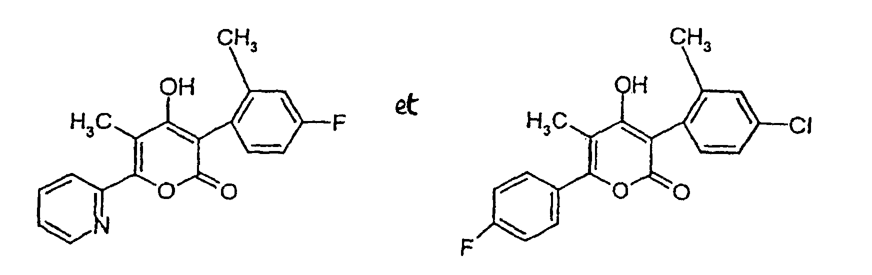
étant exclus.

3. Composé de formule (I) suivant la revendication 1, formule dans laquelle
X est un reste méthyle ou éthyle et
Y est le fluor ou le chlore
ou bien
X est le fluor ou le chlore et
Y est un reste méthyle ou éthyle,
A représente l'hydrogène, un reste alkyle ayant 1 à 6 atomes de carbone ou un reste phényle qui peut porter un ou deux substituants, identiques ou différents, fluoro, chloro, méthyle, éthyle, n-propyle, isopropyle, trifluorométhyle, méthoxy, éthoxy, trifluorométhoxy, cyano et/ou nitro,
D est l'hydrogène, un reste alkyle ayant 1 à 8 atomes de carbone,
ou représente un reste cycloalkyle ayant 3 à 7 atomes de carbone portant éventuellement un ou deux substituants, identiques ou différents, fluoro, chloro, méthyle, éthyle, méthoxy, éthoxy et/ou trifluorométhyle,
ou représente un reste phényle qui peut porter un ou deux substituants, identiques ou différents, fluoro, chloro, bromo, méthyle, éthyle, n-propyle, isopropyle, n-butyle, sec.-butyle, isobutyle, tertio-butyle, trifluorométhyle, méthoxy, éthoxy, trifluorométhoxy, cyano, nitro, phényle et/ou phénoxy, les deux restes mentionnés en dernier lieu pouvant eux-mêmes être substitués une ou deux fois par du fluor, du chlore, un radical méthyle, méthoxy, trifluorométhyle et/ou trifluorométhoxy,
ou un reste furannyle, pyridyle ou thiényle, ces restes pouvant porter un ou deux substituants fluoro, chloro, bromo, méthyle, éthyle, n-propyle, isopropyle, trifluorométhyle, trifluorométhoxy, cyano et/ou nitro,
ou bien
D représente un reste de formule 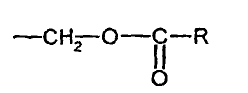 dans laquelle
R est un reste phényle qui peut porter un ou deux substituants, identiques ou différents, fluoro, chloro, méthyle, éthyle, trifluorométhyle, méthoxy, trifluorométhoxy, cyano et/ou nitro,
A et D forment en outre conjointement un groupe alcanediyle en C₃ à C₅ ou un groupe alcènediyle en C₃ à C₅, dans chacun desquels, le cas échéant, un groupe méthylène est remplacé par de l'oxygène ou du soufre et qui sont éventuellement substitués une à quatre fois identiques ou différentes par du fluor, du chlore, du brome, un radical méthyle, éthyle, n-propyle, iso-propyle, n-butyle, isobutyle, sec.- butyle, tertio-butyle, méthoxy, trifluorométhyle et/ou trifluorométhoxy ou par un autre groupe butadiènediyle formant un noyau condensé, qui peut être substitué une ou deux fois par un radical méthyle,
les composés de formules 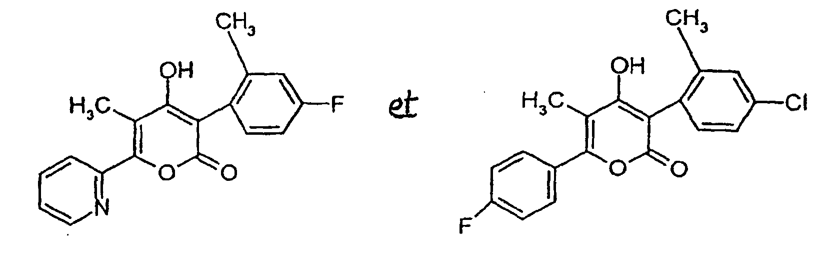 étant exclus.

4. Procédé de production de 3-phényl-pyrones de formule (I) suivant la revendication 1, **caractérisé en ce qu'**on fait réagir
α) des composés carbonyliques de formule 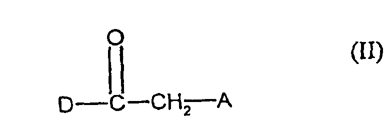 dans laquelle
A et D ont les définitions indiquées ci-dessus,
ou bien
β) des éthers de silyle de formule 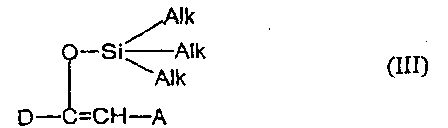 dans laquelle
A et D ont les définitions indiquées ci-dessus et
Alk désigne un reste alkyle ayant 1 à 4 atomes de carbone,
dans chaque cas avec des dérivés de cétène de formule 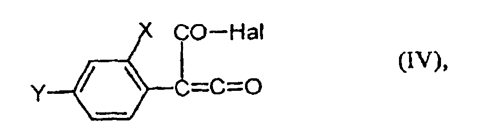 dans laquelle
Y et Y ont les définitions indiquées ci-dessus et
Hal représente le chlore ou le brome,
éventuellement en présence d'un diluant et, le cas échéant, en présence d'un accepteur d'acide.

5. Compositions pesticides, fongicides et herbicides, **caractérisées par** une teneur en au moins une 3-phénylpyrone de formule (I) suivant la revendication 1, à côté de diluants et/ou de substances tensio-actives.

6. Utilisation de 3-phényl-pyrones de formule (I) suivant la revendication 1 comme pesticides, fongicides et herbicides.

7. Procédé pour combattre des parasites animaux, **caractérisé en ce qu'**on épand des 3-phényl-pyrones de formule (I) suivant la revendication 1 sur les parasites et/ou sur leur milieu.

8. Procédé pour combattre des champignons, **caractérisé en ce qu'**on épand des 3-phényl-pyrones de formule (I) suivant la revendication 1 sur les champignons et/ou sur leur milieu.

9. Procédé pour combattre des mauvaises herbes, **caractérisé en ce qu'**on épand des 3-phényl-pyrones de formule (I) suivant la revendication 1 sur les mauvaises herbes et/ou sur leur milieu.

10. Procédé de préparation de compositions pesticides, fongicides et herbicides, **caractérisé en ce qu'**on mélange des 3-phényl-pyrones de formule (I) suivant la revendication 1 avec des diluants et/ou des agents tensioactifs.
